# EUROPEAN PATENT APPLICATION

(11) **EP 4 506 458 A1**
(43) Date of publication of application: **12.02.2025**
(21) Application number: 23784819.7
(22) Date of filing: 07.04.2023
(51) Int. Cl.: C12N 15/62, A61K 38/49, A61K 39/395, A61K 47/68, A61P 7/02, A61P 9/00, C07K 14/745, C07K 16/18, C07K 19/00, C12N 9/72, C12N 15/13, C12N 15/54, C12N 15/58, C12P 21/02

(54) **ANTIBODY BINDING TO FIBRIN, FIBRINOLYTIC PROTEIN CONTAINING ANTIBODY, AND PHARMACEUTICAL FORMULATION CONTAINING PROTEIN**

(30) Priority: 08.04.2022 JP 2022064495; 05.10.2022 JP 2022160686
(71) Applicant: RIN Institute Inc., Tokyo, 104-0045 (JP)
(72) Inventor: MATSUMURA, Yasuhiro, Tokyo 104-0045 (JP); HANAOKA, Shingo, Tokyo 104-0045 (JP)
(74) Representative: Simmons & Simmons LLP (Munich)
(86) International application number: PCT/JP2023/014377
(87) International publication number: WO 2023/195535

(57) **Abstract**

The present invention provides: a stable fibrin clot-soluble protein excellent in production in protein-expressing cells in a serum-free medium and suppressing the production of impurities such as degradation products; a pharmaceutical formulation containing the protein; and a production method for the protein and pharmaceutical formulation.

## Description

### Technical Field

The present invention relates to an antibody that binds to fibrin, a fibrinolytic protein containing the antibody and a pharmaceutical formulation containing the protein. The present invention particularly relates to a fibrinolytic protein suitable for large-scale purification and a pharmaceutical formulation containing the protein. The present invention also relates to a method for stabilizing a fibrinolytic protein and a method for producing a stabilized fibrinolytic protein.

### Background Art

Antibodies binding to fibrin with a stronger affinity than to fibrinogen have been developed (Patent Literatures 1 and 2). These Patent Literatures disclose that the antibodies are used for treating cancer. The antibodies that strongly binds to fibrin may be used for *in-vivo* imaging (Patent Literatures 1 and 2).

Furthermore, it is disclosed that a fusion protein of an antibody that binds to fibrin and a prourokinase mutant has been developed and has thrombolytic activity (Patent Literature 3).

### Citation List

### Patent Literatures

Patent Literature 1: WO2014/133093
Patent Literature 2: WO2018/203517
Patent Literature 3: WO2021/200922

### Summary of Invention

The present invention provides an antibody that binds to fibrin, a fibrinolytic protein containing the antibody and a pharmaceutical formulation containing the protein. To describe more specifically, problems of large-scale production in WO2021/200922 (Patent Literature 3) are revealed as follows: for example, an increase of production by protein-producing cells has an upper limit; a degradation product of a protein produced is generated; serum is required for culture, a predetermined additive is required for purification, and inadvertent activation). According to an embodiment of the present invention, at least one of the above problems can be reduced or solved. Furthermore, the fusion protein disclosed in Patent Literature 3 did not bind to a mouse urokinase receptor but exhibited reactivity to a human urokinase receptor. According to an embodiment of the present invention, the reactivity to a human urokinase receptor can be reduced or eliminated.

According to the present invention, the following inventions are provided.
[1] A fusion protein comprising an antibody that binds to insoluble fibrin or an antigen-binding fragment thereof and a catalytic domain of prourokinase having an amino acid sequence corresponding to a sequence of amino acids at positions 156 to 411 of prourokinase set forth in SEQ ID NO: 6 or 7, in which the antibody or an antigen-binding fragment thereof and the catalytic domain of prourokinase are linked directly or via a linker to each other, wherein
   the antibody or an antigen-binding fragment thereof contains a heavy chain variable region and light chain variable region of the antibody that binds to insoluble fibrin; and
   phenylalanine of the catalytic domain corresponding to phenylalanine at position 157 of the prourokinase set forth in SEQ ID NO: 6 or 7 is substituted with another amino acid.
[2] The fusion protein according to item [1], wherein the phenylalanine is substituted with isoleucine, glutamic acid, aspartic acid or cysteine.
[3] The fusion protein according to item [1] or [2], wherein the phenylalanine is substituted with glutamic acid or aspartic acid.
[4] The fusion protein according to any one of items [1] to [3], wherein
   binding ability to at least one urokinase receptor selected from the group consisting of a human urokinase receptor and a mouse urokinase receptor is lower than binding ability of a fusion protein having an amino acid sequence set forth in SEQ ID NO: 1.
[5] The fusion protein according to any one of items [1] to [4], wherein
   the fusion protein shows no significant binding to one or more urokinase receptor selected from the group consisting of a human urokinase receptor and a mouse urokinase receptor.
[6] The fusion protein according to any one of items [1] to [3], further comprising
   an EGF-like domain having an amino acid sequence corresponding to a sequence of amino acids at positions 7 to 43 of the prourokinase set forth in SEQ ID NO: 6 or 7 between the antibody or an antigen-binding fragment portion thereof and the catalytic domain, wherein
   amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are each substituted with another amino acid, thereby lowering or eliminating binding ability to a human urokinase receptor.
[7] The fusion protein according to any one of items [1] to [4], further comprising
   an EGF-like domain having an amino acid sequence corresponding to a sequence of amino acids at positions 7 to 43 of the prourokinase set forth in SEQ ID NO: 6 or 7 between the antibody or an antigen-binding fragment portion thereof and the catalytic domain, wherein
   amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating binding ability to a human urokinase receptor.
[8] The fusion protein according to any one of items [1] to [4], further comprising
   an EGF-like domain having an amino acid sequence corresponding to a sequence of amino acids at positions 7 to 43 of the prourokinase set forth in SEQ ID NO: 6 or 7 between the antibody or an antigen-binding fragment portion thereof and the catalytic domain, wherein
   amino acids of the EGF-like domain corresponding to the amino acids from asparagine at position 22 to isoleucine at position 28 of the prourokinase set forth in SEQ ID NO: 6 or 7 are deleted.
[9] A pharmaceutical formulation comprising the fusion protein according to any one of items [1] to [8].
[10] A method for producing the fusion protein according to any one of items [1] to [8], comprising culturing a protein-producing cell expressibly having a nucleic acid encoding the fusion protein to express the fusion protein and purifying the fusion protein from a culture obtained.
[11] The method according to item [10], wherein the culturing is performed in a serum-free condition or in the presence of 5% or less of serum.
[12] The method according to item [10] or [11], wherein the purifying is performed in the absence of L-arginine.
[13] A method for producing a fusion-protein variant preferably excellent in stability or a pharmaceutical composition containing the fusion-protein variant, wherein
   the fusion protein comprises
   an antibody that binds to insoluble fibrin or an antigen-binding fragment thereof and a catalytic domain of prourokinase having an amino acid sequence corresponding to a sequence of amino acids at positions 156 to 411 of prourokinase set forth in SEQ ID NO: 6 or 7, in which the antibody or an antigen-binding fragment thereof and the catalytic domain of prourokinase are linked directly or via a linker to each other;
   the antibody or antigen-binding fragment thereof contains a heavy chain variable region and light chain variable region of the antibody that binds to insoluble fibrin; and
   the method comprising introducing an F157E or F157D mutation to the fusion protein, thereby imparting excellent stability compared to the fusion protein not modified.
[21] A fusion protein comprising an antibody that binds to insoluble fibrin or an antigen-binding fragment thereof and a catalytic domain of prourokinase having an amino acid sequence corresponding to a sequence of the amino acids at positions 156 to 411 of the prourokinase set forth in SEQ ID NO: 6 or 7, in which the antibody or an antigen-binding fragment thereof and the catalytic domain of prourokinase are linked directly or via a linker to each other, wherein
   the antibody or an antigen-binding fragment thereof contains a heavy chain variable region and light chain variable region of the antibody that binds to insoluble fibrin; an EGF-like domain having an amino acid sequence corresponding to the sequence of the amino acids at positions 7 to 43 of the prourokinase set forth in SEQ ID NO: 6 or 7 is further contained between the antibody or an antigen-binding fragment portion thereof and the catalytic domain; and the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are each substituted with another amino acid, thereby lowering or eliminating the binding ability to a human urokinase receptor.
[22] A fusion protein comprising an antibody that binds to insoluble fibrin or an antigen-binding fragment thereof and a catalytic domain of prourokinase having an amino acid sequence corresponding to a sequence of the amino acids at positions 156 to 411 of the prourokinase set forth in SEQ ID NO: 6 or 7, in which the antibody or an antigen-binding fragment thereof and the catalytic domain of prourokinase are linked directly or via a linker to each other, wherein
   the antibody or an antigen-binding fragment thereof contains a heavy chain variable region and light chain variable region of the antibody that binds to insoluble fibrin;
   an EGF-like domain having an amino acid sequence corresponding to a sequence of the amino acids at positions 7 to 43 of the prourokinase set forth in SEQ ID NO: 6 or 7 is further contained between the antibody or an antigen-binding fragment portion thereof and the catalytic domain; and the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating the binding ability to a human urokinase receptor.
[23] A fusion protein comprising an antibody that binds to insoluble fibrin or an antigen-binding fragment thereof, an EGF-like domain of prourokinase having an amino acid sequence corresponding to the amino acids at positions 7 to 43 of the prourokinase set forth in SEQ ID NO: 6 or 7, a kringle domain of prourokinase corresponding to the amino acids at positions 49 to 131 thereof, and a catalytic domain of prourokinase having an amino acid sequence corresponding to the sequence of the amino acids at positions 156 to 411 thereof, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin;
   an EGF-like domain having an amino acid sequence corresponding to the sequence of the amino acids at positions 7 to 43 of the prourokinase set forth in SEQ ID NO: 6 or 7 is further contained between the antibody or an antigen-binding fragment portion thereof and the catalytic domain; and the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are each substituted with another amino acid, thereby lowering or eliminating the binding ability to a human urokinase receptor.
[24] A fusion protein comprising an antibody that binds to insoluble fibrin or an antigen-binding fragment thereof, an EGF-like domain of prourokinase having an amino acid sequence corresponding to amino acids at positions 7 to 43 of the prourokinase set forth in SEQ ID NO: 6 or 7, a kringle domain of prourokinase corresponding to the amino acids at positions 49 to 131, and a catalytic domain of prourokinase having an amino acid sequence corresponding to the sequence of the amino acids at positions 156 to 411, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin;
   an EGF-like domain having an amino acid sequence corresponding to a sequence of the amino acids at positions 7 to 43 of the prourokinase set forth in SEQ ID NO: 6 or 7 is further contained between the antibody or an antigen-binding fragment portion thereof and the catalytic domain; and the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating the binding ability to a human urokinase receptor.
[25] A fusion protein comprising an antibody that binds to insoluble fibrin or an antigen-binding fragment thereof, an EGF-like domain of prourokinase having an amino acid sequence corresponding to the amino acids at positions 7 to 43 of the prourokinase set forth in SEQ ID NO: 6 or 7, a kringle domain of prourokinase corresponding to the amino acids at positions 49 to 131, and a catalytic domain of prourokinase having an amino acid sequence corresponding to the sequence of the amino acids at positions 156 to 411, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin;
   an EGF-like domain having an amino acid sequence corresponding to the sequence of the amino acids at positions 7 to 43 of the prourokinase set forth in SEQ ID NO: 6 or 7 is further contained between the antibody or an antigen-binding fragment portion thereof and the catalytic domain; and the amino acid sequence of the EGF-like domain corresponding to the amino acid sequence from asparagine at position 22 to isoleucine at position 28 of prourokinase set forth in SEQ ID NO: 6 or 7 is deleted, thereby lowering or preferably eliminating the binding ability to a human urokinase receptor.
[26] A fusion protein comprising an antibody that binds to insoluble fibrin or an antigen-binding fragment thereof, an EGF-like domain of prourokinase having an amino acid sequence corresponding to the amino acids at positions 7 to 43 of the prourokinase set forth in SEQ ID NO: 6 or 7, a kringle domain of prourokinase corresponding to the amino acids at positions 49 to 131, and a catalytic domain of prourokinase having an amino acid sequence corresponding to the sequence of the amino acids at positions 156 to 411, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin;
   an EGF-like domain having an amino acid sequence corresponding to the sequence of the amino acids at positions 7 to 43 of the prourokinase set forth in SEQ ID NO: 6 or 7 is further contained between the antibody or an antigen-binding fragment portion thereof and the catalytic domain; the amino acid sequence of the EGF-like domain corresponding to the amino acid sequence from asparagine at position 22 to isoleucine at position 28 of prourokinase set forth in SEQ ID NO: 6 or 7 is deleted, thereby lowering or preferably eliminating the binding ability to a human urokinase receptor; and 1 to 7 amino acids (a peptide having no binding affinity to the human urokinase receptor, for example, GG) are inserted into the deletion site, thereby lowering or preferably eliminating the binding ability to a human urokinase receptor.
[31A] A fusion protein comprising an antibody that binds to insoluble fibrin or an antigen-binding fragment thereof and a catalytic domain of prourokinase having an amino acid sequence corresponding to the sequence of the amino acids at positions 156 to 411 of the prourokinase set forth in SEQ ID NO: 6 or 7, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin; and
   phenylalanine of the catalytic domain corresponding to phenylalanine at position 157 of the prourokinase set forth in SEQ ID NO: 6 or 7 is substituted with another amino acid.
[31B] A fusion protein comprising
   an antibody that binds to insoluble fibrin or an antigen-binding fragment thereof,
   an EGF-like domain of prourokinase having an amino acid sequence corresponding to the amino acids at positions 7 to 43 of the prourokinase set forth in SEQ ID NO: 6 or 7, and
   a catalytic domain of prourokinase having an amino acid sequence corresponding to the sequence of the amino acids at positions 156 to 411 of the prourokinase set forth in SEQ ID NO: 6 or 7, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin; and
   phenylalanine of the catalytic domain corresponding to phenylalanine at position 157 of the prourokinase set forth in SEQ ID NO: 6 or 7 is substituted with another amino acid.
[31C] A fusion protein comprising an antibody that binds to insoluble fibrin or an antigen-binding fragment thereof, a kringle domain of prourokinase corresponding to the amino acids at positions 49 to 131 of the prourokinase set forth in SEQ ID NO: 6 or 7, and
   a catalytic domain of prourokinase having an amino acid sequence corresponding to the sequence of the amino acids at positions 156 to 411, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin; and phenylalanine of the catalytic domain corresponding to phenylalanine at position 157 of the prourokinase set forth in SEQ ID NO: 6 or 7 is substituted with another amino acid.
[31D] A fusion protein comprising
   an antibody that binds to insoluble fibrin or an antigen-binding fragment thereof,
   an EGF-like domain of prourokinase having an amino acid sequence corresponding to the amino acids at positions 7 to 43 of the prourokinase set forth in SEQ ID NO: 6 or 7, a kringle domain of prourokinase corresponding to the amino acids at positions 49 to 131 and a catalytic domain of prourokinase having an amino acid sequence corresponding to the sequence of the amino acids at positions 156 to 411 of the prourokinase set forth in SEQ ID NO: 6 or 7, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin; and
   phenylalanine of the catalytic domain corresponding to phenylalanine at position 157 of the prourokinase set forth in SEQ ID NO: 6 or 7 is substituted with another amino acid.
[32] The fusion protein according to item [31] (more specifically, any one of [31A] to [31D]), wherein the phenylalanine is substituted with isoleucine, glutamic acid, aspartic acid or cysteine.
[33] The fusion protein according to item [31] or [32], wherein the phenylalanine is substituted with glutamic acid or aspartic acid.
[34] The fusion protein according to any one of items [31] to [33], further comprising the EGF-like domain having an amino acid sequence corresponding to the sequence of the amino acids at positions 7 to 43 of the prourokinase set forth in SEQ ID NO: 6 or 7 between the antibody or an antigen-binding fragment portion thereof and the catalytic domain, wherein the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are each substituted with another amino acid, thereby lowering or eliminating the binding ability to a human urokinase receptor.
[35] The fusion protein according to any one of items [31] to [34], further comprising an EGF-like domain having an amino acid sequence corresponding to the sequence of the amino acids at positions 7 to 43 of the prourokinase set forth in SEQ ID NO: 6 or 7 between the antibody or an antigen-binding fragment portion thereof and the catalytic domain, wherein the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating the binding ability to a human urokinase receptor.
[36] The fusion protein according to any one of items [31] to [34], further comprising the EGF-like domain having an amino acid sequence corresponding to the sequence of the amino acids at positions 7 to 43 of the prourokinase set forth in SEQ ID NO: 6 or 7 between the antibody or an antigen-binding fragment portion thereof and the catalytic domain, wherein the amino acid sequence of the EGF-like domain corresponding to the amino acid sequence from asparagine at position 22 to isoleucine at position 28 of prourokinase set forth in SEQ ID NO: 6 or 7 is deleted (for example, at the deletion site, 1 to 7 amino acids are inserted (a peptide having no binding affinity at least to a human urokinase receptor, preferably, a peptide having no binding affinity to a human urokinase receptor and a mouse urokinase receptor, for example, a peptide consisting of two amino acids such as GG)), thereby lowering or preferably eliminating the binding ability to a human urokinase receptor.
[37] A pharmaceutical formulation comprising the fusion protein according to any one of items [31] to [36] .
[38] A method for producing the fusion protein according to any one of items [31] to [36], comprising
   culturing a protein-producing cell expressibly having a nucleic acid encoding the fusion protein to express the fusion protein and purifying the fusion protein from the culture to be obtained.
[39] The method according to item [38], wherein the culture is performed in a serum-free condition or in the presence of 5% or less of serum.
[40] The method according to item [38] or [39], wherein purification is performed in the absence of L-arginine.
[41] A fusion protein comprising an antibody that binds to insoluble fibrin or an antigen-binding fragment thereof, an EGF-like domain of prourokinase having an amino acid sequence corresponding to the amino acids at positions 7 to 43 of the prourokinase set forth in SEQ ID NO: 6 or 7, a kringle domain of prourokinase corresponding to the amino acids at positions 49 to 131, and a catalytic domain of prourokinase having an amino acid sequence corresponding to the sequence of the amino acids at positions 156 to 411, in which the antibody or an antigen-binding fragment thereof and the catalytic domain of prourokinase are linked directly or via a linker to each other, wherein
   the linker is a non-cleavable linker;
   the antibody or an antigen-binding fragment thereof contains a heavy chain variable region and light chain variable region of the antibody that binds to insoluble fibrin;
   phenylalanine of the catalytic domain corresponding to phenylalanine at position 157 of the prourokinase set forth in SEQ ID NO: 6 or 7 is substituted with another amino acid; and
   the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are each substituted with another amino acid, thereby lowering or eliminating the binding ability to a human urokinase receptor.
[42] The fusion protein according to item [41], wherein phenylalanine of the catalytic domain corresponding to phenylalanine at position 157 is substituted with another amino acid except for tyrosine.
[43] The fusion protein according to item [41], wherein phenylalanine of the catalytic domain corresponding to phenylalanine at position 157 is substituted with isoleucine, glutamic acid, aspartic acid or cysteine.
[44] The fusion protein according to item [41], wherein phenylalanine of the catalytic domain corresponding to phenylalanine at position 157 is glutamic acid or aspartic acid.
[45] The fusion protein according to item [41], wherein the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating the binding ability to a human urokinase receptor.
[46] The fusion protein according to item [42], wherein the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating the binding ability to a human urokinase receptor.
[47] The fusion protein according to item [43], wherein the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating the binding ability to a human urokinase receptor.
[48] The fusion protein according to item [44], wherein the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating the binding ability to a human urokinase receptor.
[61] The fusion protein according to any one of the above items, wherein the antibody or an antigen-binding fragment thereof specifically binds to insoluble fibrin (particularly binds, with lower affinity than to the insoluble fibrin, and particularly preferably does not significantly bind, to at least one, preferably all selected from the group consisting of fibrinogen, soluble fibrin and a fibrin degradation product).
[62] The fusion protein according to any one of the above items, wherein the antibody or an antigen-binding fragment thereof binds to a peptide consisting of the amino acid sequence set forth in SEQ ID NO: 8.
[63] The fusion protein according to any one of the above items, wherein
   the antibody or an antigen-binding fragment thereof has heavy chain CDR1 to 3 and light chain CDR1 to 3 of an antibody selected from the group consisting of 102-10 antibody, 34-105 antibody and Fib-0355 antibody disclosed in WO2014/133093, as the corresponding CDRs, respectively.
[64] The fusion protein according to any one of the above items, wherein the antibody or an antigen-binding fragment thereof comprises
   a heavy chain variable region containing heavy chain CDR1 having the amino acid sequence set forth in SEQ ID NO: 10, heavy chain CDR 2 having the amino acid sequence set forth in SEQ ID NO: 11, and heavy chain CDR 3 having the amino acid sequence set forth in SEQ ID NO: 12 and
   a light chain variable region containing light chain CDR1 having the amino acid sequence set forth in SEQ ID NO: 13, light chain CDR2 having the amino acid sequence set forth in SEQ ID NO: 14 and light chain CDR3 having the amino acid sequence set forth in SEQ ID NO: 15.
[65] The fusion protein according to any one of the above items, wherein the antibody or an antigen-binding fragment thereof comprises
   a heavy chain variable region having the amino acid sequence set forth in SEQ ID NO: 16 and
   a light chain variable region having the amino acid sequence set forth in SEQ ID NO: 17.
[66] The fusion protein according to any one of the above items, comprising
   a heavy chain variable region containing heavy chain CDR1 having the amino acid sequence set forth in SEQ ID NO: 18, heavy chain CDR 2 having the amino acid sequence set forth in SEQ ID NO: 19, and heavy chain CDR 3 having the amino acid sequence set forth in SEQ ID NO: 20; and
   a light chain variable region containing light chain CDR1 having the amino acid sequence set forth in SEQ ID NO: 21, light chain CDR2 having the amino acid sequence set forth in SEQ ID NO: 22 and light chain CDR3 having the amino acid sequence set forth in SEQ ID NO: 23.
[67] The fusion protein according to any one of the above items, wherein the antibody or an antigen-binding fragment thereof comprises
   a heavy chain variable region having the amino acid sequence set forth in SEQ ID NO: 24 and
   a light chain variable region having the amino acid sequence set forth in SEQ ID NO: 25.
[68] The fusion protein according to any one of the above items, wherein the antibody or an antigen-binding fragment thereof competes with a reference antibody for binding to insoluble fibrin and the reference antibody comprises
   a heavy chain variable region having the amino acid sequence set forth in SEQ ID NO: 16 and
   a light chain variable region having the amino acid sequence set forth in SEQ ID NO: 17.
[69] The fusion protein according to any one of the above items, wherein the antibody or an antigen-binding fragment thereof competes with a reference antibody for binding to insoluble fibrin and the reference antibody comprises
   a heavy chain variable region having the amino acid sequence set forth in SEQ ID NO: 24 and
   a light chain variable region having the amino acid sequence set forth in SEQ ID NO: 25.
[70] The fusion protein according to any one of the above items, having the amino acid sequence set forth in SEQ ID NO: 107.
[71] The fusion protein according to any one of the above items, wherein the antibody or an antigen-binding fragment thereof has a binding dissociation constant (KD) of 10⁻⁶ M or less, 10⁻⁷ M or less or 10⁻⁸ M or less.
[72] The fusion protein according to any one of the above items, wherein the antibody or an antigen-binding fragment thereof is a Fab fragment or a Fab' fragment {wherein preferably, the fusion protein can be a fusion protein formed by linking the C terminal of a heavy chain of the fragment and the N terminal of prourokinase or a portion or a mutant thereof}.
[81] The fusion protein according to any one of the above items, having 1 or more, preferably 2 or more, more preferably 3 or more, further preferably all selected from the group consisting of the properties: (i) binding specificity to insoluble fibrin; (ii) binding affinity to insoluble fibrin; (iii) resistance to inhibition by a plasminogen activator inhibitor (PAI-1); and (iv) low inducibility of matrix metalloproteinase (MMP) activity in the tissue around of thrombi.
[82] The fusion protein according to any one of the above items, linked to an imaging agent.
[91] A pharmaceutical formulation comprising the fusion protein according to any one of the above items.
[92] The pharmaceutical formulation according to item [91] for treating thrombosis.
[93] The pharmaceutical formulation according to item [91] for treating a condition or disease for which dissolving fibrin clots is suitable.
[94] The pharmaceutical formulation according to item [91], for treating a thrombogenesis-associated cerebrovascular disorder and cardiovascular disorder.
[95] The pharmaceutical formulation according to item [91], for treating a condition or symptom caused by thrombogenesis in a cerebrovascular disorder and a cardiovascular disorder.
[96] The pharmaceutical formulation according to item [91], for treating a condition or symptom of a cerebrovascular disorder and a cardiovascular disorder.
[97] The pharmaceutical formulation according to item [91], for treating ischemic cerebrovascular disorder.
[98] The pharmaceutical formulation according to item [91], for treating either one of cerebral infarction and transient ischemic attack.
[99] The pharmaceutical formulation according to item [91], for treating ischemic cardiovascular disorder.
[100] The pharmaceutical formulation according to item [91], for treating either one of cardiac angina and myocardial infarction.
[101] The pharmaceutical formulation according to any one of items [91] to [100] for treating a disease in an acute phase thereof.
[102] The pharmaceutical formulation according to item [91], for dissolving fibrin clots in a subject having fibrin clots.
[103] A pharmaceutical formulation for detecting thrombi *in vivo,* comprising the fusion protein according to item [82].
[104] The pharmaceutical formulation according to item [103] simultaneously applied for medical use defined in any one of the above [92] to [102].

### Brief Description of Drawings

[Figure 1] Figure 1 shows the reactivities of AMU1114 variants (particularly a variant of F157 in urokinase moiety) to a substrate. The vertical axis represents absorbance at 405 nm.
[Figure 2] Figure 2 shows the reactivities of AMU1114 variants (particularly a variant of F157 in urokinase moiety) treated with plasmin to a substrate. The vertical axis represents absorbance at 405 nm.
[Figure 3A] Figure 3A shows the electrophoresis results of SDS-PAGE of AMU1114 variants (particularly a variant of F157 in urokinase moiety) incubated in PBS of 37°C for 0 hours.
[Figure 3B] Figure 3B shows the electrophoresis results of SDS-PAGE of AMU1114 variants (particularly a variant of F157 in urokinase moiety) incubated in PBS of 37°C for 24 hours.
[Figure 3C] Figure 3C shows the electrophoresis results of SDS-PAGE of AMU1114 variants (particularly a variant of F157 in urokinase moiety) incubated in PBS of 37°C for 120 hours.
[Figure 4] Figure 4 shows the reactivities of AMU1114 variants treated with plasmin to a substrate. The vertical axis represents absorbance at 405 nm.
[Figure 5] Figure 5 shows the reactivities of AMU1114 variants to a human urokinase receptor or a mouse urokinase receptor to a substrate. The vertical axis represents absorbance at 405 nm.
[Figure 6A] Figure 6A shows a gel-filtration chromatogram of urokinase expressed in Chinese hamster ovary (CHO) cells in the presence of different concentrations of fetal bovine serum (FBS) and purified.
[Figure 6B] Figure 6B shows electrophoresis results of urokinase expressed in Chinese hamster ovary (CHO) cells in the presence of different concentrations of fetal bovine serum (FBS) and purified.
[Figure 7A] Figure 7A shows electrophoresis results of AMU1114 expressed in Chinese hamster ovary (CHO) cells in the presence or absence of 20% FBS and purified.
[Figure 7B] Figure 7B shows electrophoresis results of AMU1114 variants expressed in Chinese hamster ovary (CHO) cells in the absence of serum (in serum-free medium) and purified.
[Figure 8A] Figure 8A shows a gel-filtration chromatogram of AMU1114 expressed in Chinese hamster ovary (CHO) cells in the absence of serum (in serum-free medium) and purified.
[Figure 8B] Figure 8B shows a gel-filtration chromatogram of AMU1114 variants expressed in Chinese hamster ovary (CHO) cells in the absence of serum (in serum-free medium) and purified.
[Figure 9] Figure 9 shows the alignments of AMU1114 and variants thereof.
[Figure 10] Figure 10 shows the reactivity of AMU1114 variants treated with plasmin to a substrate. The vertical axis represents absorbance at 405 nm.
[Figure 11] Figure 11 shows the reactivities of AMU1114 variants to a substrate to a human urokinase receptor or a mouse urokinase receptor. The vertical axis represents absorbance at 405 nm.
[Figure 12A] Figure 12A shows the histological stain images of the lungs of thrombin thrombus model mice administered with PBS, AMU1114 (Δ22-28, F157E) (0.08 mg/kg weight or 0.32 mg/kg weight; represented by Amu 0.08 and Amu 0.32, respectively) or tenecteplase (0.05 mg/kg weight or 0.2 mg/kg weight; represented by Tene0.05 and Tene0.2, respectively).
[Figure 12B] Figure 12B is a graph showing the total area of signals corresponding to thrombi.

### Detailed Description of the Invention

In the specification, the term "subject" is a mammal. The mammal may be a dog, a cat, a cow, a horse, a pig, a primate (for example, a monkey, a gorilla, an orangutan, a bonobo, a chimpanzee and a human), and more specifically, a human.

In the specification, the term "fibrin" refers to an insoluble coagulate formed by cleaving the C terminal of fibrinogen constituted of three types of polypeptide chains (Aα chain, Bβ chain and γ chain). In the specification, fibrin will be sometimes referred to as insoluble fibrin. More specifically, when the C terminal of fibrinogen is cleaved, fibrinogen is converted into the state called a fibrin monomer. The fibrin monomers are polymerized by the action of calcium to form a poor-soluble fibrin polymer. The fibrin polymers are crosslinked between the polymers by the action of factor XIII to form stable fibrin (insoluble fibrin defined in the specification or fibrin gel). Insoluble fibrin is decomposed by plasmin. Plasmin is contained in the plasma in the form of a precursor, plasminogen. When plasminogen is decomposed with, e.g., a plasminogen activator (for example, urokinase, tissue plasminogen activator and streptokinase), more specifically, when the peptide between Arg and Val of plasminogen is decomposed, plasmin is produced. Plasmin is inhibited by a protein called a plasmin inhibitor and can be limited in action.

Aα chain of fibrinogen may be Aα chain of human fibrinogen. Examples of Aα chain of human fibrinogen include Aα chain of human fibrinogen having the amino acid sequence registered under GenBank registration number: AAI01936.1, and Aα chain of human fibrinogen having an amino acid sequence corresponding to the amino acid sequence.

Bβ chain of fibrinogen may be Bβ chain of human fibrinogen. Examples of Bβ chain of human fibrinogen include β chain of human fibrinogen having the amino acid sequence registered under NCBI reference number: NP_005132.2, and β chain of human fibrinogen having an amino acid sequence corresponding to the amino acid sequence.

γ chain of fibrinogen may be γ chain of human fibrinogen. Examples of γ chain of human fibrinogen include γ chain of human fibrinogen having the amino acid sequence registered under GenBank registration number: AAH07044.1, and γ chain of human fibrinogen having an amino acid sequence corresponding to the amino acid sequence.

In the specification, "urokinase" is one of serine protease called as a urokinase-type plasminogen activator (uPA) (for example, may be an enzyme registered under EC 3.4.21.73). Urokinase is produced as a precursor thereof, that is, prourokinase. When the peptide bond between Lys158 and Ile159 is cleaved, active-type urokinase (the chains cleaved are mutually linked via a disulfide bond) is formed. Urokinase has three domains: an EGF-like domain, a kringle domain and a catalytic domain. When the bond between Lys135 and Lys136 of urokinase is cleaved, a low molecular type urokinase is produced. Note that, the positions of amino acids of prourokinase are determined based on the amino acid sequence of the prourokinase after cleavage of the signal peptide (for example, the amino acid sequence set forth in SEQ ID NO: 6). Examples of the prourokinase include human prourokinase. Examples of the human prourokinase include a human prourokinase having amino acid sequence registered under GenBank registration number: AAA61253.1 and human prourokinase having an amino acid sequence corresponding to the amino acid sequence. In the amino acid sequence registered under GenBank registration number: AAA61253.1, the amino acid sequence at positions 1 to 20 corresponds to a signal peptide. The EGF-like domain may have the sequence of prourokinase corresponding to positions 7 to 43 of the amino acid sequence set forth in SEQ ID NO: 6; the kringle domain may have the sequence of prourokinase corresponding to positions 49 to 131 of the amino acid sequence set forth in SEQ ID NO: 6; and the catalytic domain may have the sequence of prourokinase corresponding to positions 136 to 403. Any type of prourokinase can be used as long as it is activated in the form of a double strand by plasmin or kallikrein and has an ability to degrade plasminogen into plasmin.

In the specification, for the term "corresponding," when a second amino acid sequence has a sequence identity of 90% or more, 95% or more, 96% or more, 97% or more, 98% or more, or 99% or more to a first amino acid sequence, and the same function as that of the peptide having the first amino acid sequence (for example, in the case of urokinase, a protein has the ability to degrade plasminogen into plasmin), the second amino acid sequence is defined to have a sequence corresponding to the first amino acid sequence.

In the specification, the term "antibody" refers to an immunoglobulin. The antibody may be one of the isotypes, for example, IgG. The antibody may be preferably a monoclonal antibody. The antibody may be a human chimeric antibody, a humanized antibody or a human antibody. The human chimeric antibody may be prepared by replacing a constant region of a non-human antibody with that of a human antibody. A humanized antibody may be prepared by replacing 6 CDRs of a human antibody with the corresponding 6 CDRs of a non-human antibody. A human antibody may be prepared by use of an animal (for example, mouse) in which at least a heavy chain variable region of an immunoglobulin is replaced with the corresponding region of a human locus. If the constant region is derived from a non-human animal, the constant region is replaced with the amino acid sequence of a human antibody. In this manner, a human antibody can be obtained. In the specification, the antibody may be preferably a humanized antibody. In the specification, the antibody may be preferably a human antibody. An antibody produced in a cell has a signal peptide but the signal peptide is cut out when it is released from the cell. Thus, when an antibody is administered as a medical agent, a signal peptide of the antibody is not required.

In the specification, the term "CDR" refers to a complementarity determining region present in a heavy chain variable region and a light chain variable region of an antibody. Three CDRs are present in each of the heavy chain and light chain variable regions and called as CDR1, CDR2 and CDR3 in the order from the N terminal. CDRs can be determined based on, for example, the numbering scheme by Kabat et.al (Kabat, E. A. et al., Sequences of Proteins of Immunological Interest, 5th ed., 1991, Bethesda: US Dept. of Health and Human Services, PHS, NIH).

In the specification, the term "antigen-binding fragment of an antibody" refers to a fragment maintaining a binding ability to an antigen. Examples of the antigen-binding fragment include Fab, Fab', F(ab')₂, Fv, scFv (single chain Fv), diabody and sc(Fv)₂ (single chain (Fv)₂). For example, Fab can be obtained by digesting an antibody with papain. Or F(ab')₂ can be obtained by digesting an antibody with pepsin. If F(ab')₂ is further reduced, Fab' can be obtained. Other antigen-binding fragments of an antibody can be prepared by methods commonly known to those skilled in the art. In the present invention, the antigen-binding fragments of an antibody as mentioned above can be used.

In the specification, the term "fusion protein" refers to a protein formed by linking two or more peptides derived from different proteins via a peptide bond. In the specification, a fusion protein of a first peptide and a second peptide may contain a third peptide and further an additional peptide as long as the function of the invention is not significantly decreased. Alternatively, the fusion protein may contain only a first peptide and a second peptide. In the specification, a fusion protein of a first peptide and a second peptide may contain the first peptide and the second peptide in this order or different order, as long as the function of the invention is not significantly decreased. The fusion protein preferably contains a first peptide and a second peptide in this order. In a fusion protein, the two or more peptides derived from different proteins, may be linked with a linker interposed between them or without a linker, as long as the function of the invention is not significantly decreased. In the case where proteins are linked via a linker to each other, a flexible linker may be used.

### <Fusion-protein variant of the present invention>

(A) According to the present invention, there is provided
   a fusion protein comprising an antibody that binds to insoluble fibrin or an antigen-binding fragment thereof and a catalytic domain of prourokinase having an amino acid sequence corresponding to the sequence of amino acids at positions 136 to 403 of the prourokinase set forth in SEQ ID NO: 6, in which the antibody or an antigen-binding fragment thereof and the catalytic domain of prourokinase are linked directly or via a linker to each other. Herein, in an amino acid sequence corresponding to the sequence of amino acids at positions 136 to 403 of the prourokinase set forth in SEQ ID NO: 6, an amino acid sequence of at least one (preferably both) of lysine residues at positions 135 and 136 is substituted with another amino acid. Because of this, cleavage of the catalytic domain from prourokinase is suppressed. In a preferable embodiment, the amino acids corresponding to lysine residues at positions 135 and 136 of the prourokinase set forth in SEQ ID NO: 6 are both substituted with glycine residues. Accordingly, in a particularly preferable embodiment, a fusion-protein prourokinase or a portion thereof may have an amino acid sequence corresponding to the amino acid sequence of a prourokinase mutant of SEQ ID NO: 7 or a portion thereof.

In the fusion protein of the present invention, a prourokinase moiety thereof is inactive until the fusion protein binds to insoluble fibrin. When the fusion protein binds to insoluble fibrin, the prourokinase moiety is cleaved by plasmin present in the vicinity thereof to produce an active-form two-chain urokinase. The two-chain urokinase cleaves plasminogen around it to produce a large amount of plasmin. In this manner, insoluble fibrin is effectively dissolved.

According to the present invention, the fusion-protein prourokinase further has a kringle domain. Prourokinase may further have an EGF-like domain and a kringle domain. In a preferable embodiment, the fusion protein has an EGF-like domain, a kringle domain, and a catalytic domain in this order. In a more preferable embodiment, the fusion protein has a prourokinase region corresponding to the region of positions 7 to 403 of the amino acid sequence set forth in SEQ ID NO: 6 or 7. In the most preferable embodiment, a fusion protein may contain a prourokinase moiety having an amino acid sequence corresponding to the amino acid sequence set forth in SEQ ID NO: 6 or 7.

According to the present invention, the fusion protein contains an antibody that binds to insoluble fibrin or an antigen-binding fragment portion thereof. The antibody that binds to insoluble fibrin or an antigen-binding fragment portion thereof may contain a heavy chain variable region and a light chain variable region of the antibody that binds to insoluble fibrin or an antigen-binding fragment thereof.

According to the present invention, an antibody that binds to insoluble fibrin or an antigen-binding fragment portion thereof in the fusion protein may be a Fab fragment or a Fab' fragment. In an embodiment, the heavy chain variable region of the Fab fragment or Fab' fragment is linked to prourokinase or a moiety thereof, as mentioned above. In an embodiment, the light chain variable region of the Fab fragment or Fab' fragment is linked to prourokinase or a moiety thereof, as mentioned above.

In the specification, the terms "heavy chain" and "light chain" used in connection with an antigen-binding fragment mean whether the antibody fragment is derived from the "heavy chain" or "light chain" of the original antibody but does not mean large or small in molecular weight of the antigen-binding fragment.

In the fusion protein according to a preferable embodiment of the present invention, phenylalanine of the catalytic domain corresponding to phenylalanine at position 157 of the prourokinase set forth in SEQ ID NO: 6 is substituted with another amino acid.

(B) According to the present invention, there is provided
a fusion protein comprising
an antibody that binds to insoluble fibrin or an antigen-binding fragment thereof and a catalytic domain of prourokinase having an amino acid sequence corresponding to the amino acid sequence at positions 136 to 403 of the prourokinase set forth in SEQ ID NO: 7, in which the antibody or an antigen-binding fragment thereof and the catalytic domain of prourokinase are linked directly or via a linker to each other, wherein
phenylalanine of the catalytic domain corresponding to phenylalanine at position 157 of the prourokinase set forth in SEQ ID NO: SEQ ID NO: 7 is substituted with another amino acid.

(C) In a preferable embodiment, the present invention provides a fusion protein comprising
an antibody that binds to insoluble fibrin or an antigen-binding fragment thereof, and the region of prourokinase having an amino acid sequence corresponding to the amino acid sequence at positions 7 to 403 of the prourokinase set forth in SEQ ID NO: 7, in which the antibody or an antigen-binding fragment thereof and the catalytic domain of prourokinase are linked directly or via a linker to each other, wherein
phenylalanine of the catalytic domain corresponding to phenylalanine at position 157 of prourokinase set forth in SEQ ID NO: 7 is substituted with another amino acid.

In a more preferable embodiment, the region of prourokinase may contain a region of prourokinase having an amino acid sequence corresponding to an amino acid sequence at positions 1 to 411 of the prourokinase set forth in SEQ ID NO: 7.

According to the present invention, the above phenylalanine is substituted with any one of amino acids (for example, amino acid except for tyrosine). By this substitution, preferably, the fusion protein of the present invention can be improved in resistance to cleavage with a proteolytic enzyme (for example, plasmin). In a preferable embodiment, the above phenylalanine is substituted with alanine, lysine, cysteine, serine, isoleucine, glycine, glutamic acid or aspartic acid. In a more preferable embodiment, the above phenylalanine is substituted with isoleucine, glycine, glutamic acid or aspartic acid. By this substitution, at the time of expressing, purifying and/or storing the fusion protein, degradation and subsequent activation of the fusion protein can be suppressed. In a further preferable embodiment, the above phenylalanine is substituted with glutamic acid or aspartic acid. By this substitution, at the time of expressing, purifying and/or storing the fusion protein, degradation and subsequent activation of the fusion protein can be suppressed. In addition, activation in the presence of plasmin can exhibit the same plasminogen degradation activity as the fusion protein keeping phenylalanine unsubstituted. Furthermore, when the fusion protein keeping phenylalanine unsubstituted is produced from a protein expressing cell, fusion protein fragments are produced. However, if the above phenylalanine in the fusion protein is substituted with an amino acid except for tyrosine and preferably, glutamic acid or aspartic acid, even if a protein-expressing cell expressing the fusion protein is cultured in a low-serum medium (for example, only 5% or less of serum) or a serum-free medium, it is possible to inhibit an increase of the production of fragments of the fusion protein or reduce the production thereof. Furthermore, when fusion-protein keeping phenylalanine unsubstituted is produced from a protein-expressing cell, the production of the fusion protein continuously increases for 5 to 7 days and then stops increasing. In addition to this, the proportion occupied by a light chain in the produced protein increases. In contrast, if the above phenylalanine in the fusion protein is substituted with an amino acid except for tyrosine, preferably, glutamic acid or aspartic acid, the proportion occupied by the light chain in the produced protein can be reduced. Moreover, if the above phenylalanine in the fusion protein is substituted with an amino acid except for tyrosine, preferably, glutamic acid or aspartic acid, the production of the fusion protein continuously increases even beyond 7 days, with the result that the production of the fusion protein significantly increases (for example, 3 to 5 times) compared to a fusion protein keeping phenylalanine unsubstituted. It can be advantageous to reduce or avoid use of serum because the variation of protein production due to variation in the quality of serum can be suppressed and culture can be made at lower cost.

Accordingly, in a fusion protein, it is very significant that phenylalanine of the catalytic domain corresponding to phenylalanine at position 157 of prourokinase set forth in SEQ ID NO: 7 is substituted with another amino acid, in particular, glutamic acid or aspartic acid, in production in terms of increasing yield and purity and/or saving cost.

(D) In a preferable embodiment, the present invention provides
a fusion protein comprising
an antibody that binds to insoluble fibrin or an antigen-binding fragment thereof and a region of prourokinase having an amino acid sequence corresponding to the amino acid sequence at positions 7 to 403 of prourokinase set forth in SEQ ID NO: 7, in which the antibody or an antigen-binding fragment thereof and the catalytic domain of prourokinase are linked directly or via a linker to each other, wherein
phenylalanine of the catalytic domain corresponding to phenylalanine at position 157 of prourokinase set forth in SEQ ID NO: 7 is substituted with glutamic acid or aspartic acid.

In a preferable embodiment, the region of prourokinase has an amino acid sequence corresponding to the amino acid sequence at positions of 1 to 411 of prourokinase set forth in SEQ ID NO: 7.

### <Another fusion-protein variant of the present invention>

According to the present invention, there is provided a fusion-protein variant having a lower binding affinity to an urokinase receptor than the above fusion protein.

More specifically, in another variant of the present invention, in the amino acid sequence of any one of the above fusion proteins (for example, any one of (A) to (D)), the amino acid of the EGF-like domain corresponding to tryptophan at position 30 of the prourokinase set forth in SEQ ID NO: 6 or 7 is substituted with another amino acid (particularly arginine). By this substitution, another variant of the present invention has a lower binding affinity to a human urokinase receptor than the above fusion protein.

Furthermore, in another variant of the present invention, in the amino acid sequence of any one of the above fusion proteins (for example, any one of (A) to (D)), the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are each substituted with another amino acid. By this substitution, another variant of the present invention has a lower binding affinity to a human urokinase receptor than the above fusion protein.

According to an embodiment, in another variant of the present invention, in the amino acid sequence of any one of the above fusion proteins (for example, any one of (A) to (D)), the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively. By this substitution, another variant of the present invention has a lower binding affinity to a human urokinase receptor than the above fusion protein.

According to an embodiment, in another variant of the present invention, in the amino acid sequence of the above fusion protein (A), the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively. By this substitution, another variant of the present invention has a lower binding affinity to a human urokinase receptor than the above fusion protein.

According to an embodiment, in another fusion-protein variant of the present invention, in the amino acid sequence of the above fusion protein (B), the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively. By this substitution, another variant of the present invention has a lower binding affinity to a human urokinase receptor than the above fusion protein.

According to an embodiment, in another variant of the present invention, in the amino acid sequence of the above fusion protein (C), the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively. By this substitution, another variant of the present invention has a lower binding affinity to a human urokinase receptor than the above fusion protein.

According to an embodiment, in another variant of the present invention, in the amino acid sequence of the above fusion protein (D), the amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively. By this substitution, another variant of the present invention has a lower binding affinity to a human urokinase receptor than the above fusion protein.

According to an embodiment, in another variant of the present invention, in the amino acid sequence of any one of the above fusion proteins (for example, any one of (A) to (D)), the amino acid sequence of the EGF-like domain corresponding to the amino acid sequence from asparagine at position 22 to isoleucine at position 28 of prourokinase set forth in SEQ ID NO: 6 or 7 is deleted. By this deletion, it can be considered that the variant loses the binding ability to an urokinase receptor. According to an embodiment, in another variant of the present invention, in the amino acid sequence of any one of the above fusion proteins (for example, any one of (A) to (D)), the amino acid sequence of the EGF-like domain corresponding to the amino acid sequence from asparagine at position 22 to isoleucine at position 28 of prourokinase set forth in SEQ ID NO: 6 or 7 is deleted, and 1 to 7 amino acids (a peptide having no binding affinity to at least a human urokinase receptor, preferably, a peptide having no binding affinity to a human urokinase receptor and a mouse urokinase receptor, for example, a peptide consisting of two amino acids such as GG) are inserted in the deletion site. The amino acid sequence is inserted to compensate for a reduction in length of a protein by deletion. The amino acid sequence to be inserted is not necessary to have a special activity and may consist of or contain neutral amino acids such as G and S. By this manipulation, another variant of the present invention does not have a significant binding affinity to a human urokinase receptor (particularly, a human urokinase receptor and a mouse urokinase receptor) or has a lower binding affinity than to the receptor of the above fusion protein, preferably, does not have a significant binding affinity to a human urokinase receptor and a mouse urokinase receptor.

The binding affinity to a urokinase receptor of e.g., a human, can be 1/2 or less, 1/3 or less, 1/4 or less, 1/5 or less, 1/6 or less, 1/7 or less, 1/8 or less, 1/9 or less, 1/10 or less, 1/20 or less, 1/30 or less, 1/40 or less, 1/50 or less, 1/60 or less, 1/70 or less, 1/80 or less, 1/90 or less, or 1/100 or less, compared to that of the fusion protein set forth in SEQ ID NO: 1. The binding affinity can be determined by measuring the binding amount of a fusion protein onto a plate having a urokinase receptor immobilized thereon in accordance with ELISA assay and can be obtained by subtracting the value (background) of a negative control (for example, phosphate buffered saline (PBS)). In the ELISA assay, the binding amount of the fusion protein to a urokinase receptor can be detected by an enzyme-labeled antibody. As the enzyme-labeled antibody, an antibody (for example, an anti-human heavy chain or light chain antibody) that binds to a fusion protein can be used. As the enzyme, horseradish peroxidase (HRP) can be used. As the substrate, 3,3',5,5'-tetramethylbenzidine (TMB) can be used.

Because of this, even if the fusion protein of the present invention leaks around a thrombus, the fusion protein does not bind to a urokinase receptor in the peripheral tissue. Thus, the production of MMP enzyme in the tissue can be inhibited to prevent tissue damage. By this mechanism, it is conceivable that undesirable side effects resulting from binding of the fusion protein to an urokinase receptor can be prevented.

In a fusion-protein variant of the present invention, the antibody or an antigen-binding fragment portion thereof binds to insoluble fibrin. In a preferable embodiment, in a fusion-protein variant of the present invention, an antibody or an antigen-binding fragment portion thereof specifically binds to insoluble fibrin. The phrase "specifically binds to insoluble fibrin" means that the antibody binds to insoluble fibrin, but binds, with a lower affinity than to the insoluble fibrin, and particularly preferably does not significantly bind, to at least one, preferably all selected from the group consisting of fibrinogen, soluble fibrin and a fibrin degradation product.

In the present invention, the antibody that binds to insoluble fibrin is an antibody that binds to insoluble fibrin with a stronger affinity than to fibrinogen (in other words, an antibody that binds to insoluble fibrin with low dissociation constant K_{D} than to fibrinogen). The antibody that binds to insoluble fibrin with a stronger affinity than to fibrinogen may be, for example, an antibody that binds to a peptide consisting of the amino acid sequence set forth in SEQ ID NO: 8 or 9 (see WO2014/133093). The amino acid sequence set forth in SEQ ID NO: 8 corresponds to the amino acid sequence set forth in SEQ ID NO: 1 in WO2014/133093. The amino acid sequence set forth in SEQ ID NO: 9 corresponds to the amino acid sequence set forth in SEQ ID NO: 2 in WO2014/133093. A peptide portion consisting of the amino acid sequence set forth in SEQ ID NO: 8 or 9 is not exposed from the protein surface of fibrinogen but is exposed to the protein surface only in insoluble fibrin such that the peptide portion can access to an antibody. Accordingly, an antibody that binds to the peptide portion consisting of the amino acid sequence set forth in SEQ ID NO: 8 or 9 may have a binding selectivity to insoluble fibrin.

### <Antibody or an antigen-binding fragment portion thereof>

The antibody that binds to insoluble fibrin with a stronger affinity than to fibrinogen or an antigen-binding fragment thereof is an antibody specific to insoluble fibrin (for example, an antibody that binds to insoluble fibrin and does not substantially bind to fibrinogen), for example, an antibody or antigen-binding fragment thereof having heavy chain CDR1 to 3 and light chain CDR1 to 3 of an antibody which is selected from the group consisting of antibodies disclosed in WO2014/133093, such as 102-10 antibody (having a heavy chain variable region having heavy chain CDR1 to 3 set forth in SEQ ID NOs: 27 to 29, respectively and a light chain variable region having light chain CDR1 to 3 set forth in SEQ ID NOs: 30 to 32, respectively); 34-105 antibody (having a heavy chain variable region having heavy chain CDR1 to 3 set forth in SEQ ID NOs: 33 to 35, respectively and a light chain variable region having light chain CDR1 to 3 set forth in SEQ ID NOs: 36 to 38, respectively); and Fib-0355 antibody (having a heavy chain variable region having heavy chain CDR1 to 3 set forth in SEQ ID NOs: 39 to 41, respectively and a light chain variable region having light chain CDR1 to 3 set forth in SEQ ID NOs: 42 to 44, respectively), as the corresponding CDRs.

The antibody or that binds to insoluble fibrin with a stronger affinity than to fibrinogen or an antigen-binding fragment thereof is an antibody or an antigen binding fragment thereof having heavy chain CDR1 to 3 and light chain CDR1 to 3 of an antibody which is selected from the group consisting of antibodies disclosed, for example, in WO2018/203517 such as 99 antibody (having a heavy chain variable region having heavy chain CDR1 to 3 set forth in SEQ ID NOs: 45 to 47, respectively and a light chain variable region having light chain CDR1 to 3 set forth in SEQ ID NOs: 48 to 50, respectively); 1101 antibody (having a heavy chain variable region having heavy chain CDR1 to 3 set forth in SEQ ID NOs: 51 to 53, respectively and a light chain variable region having light chain CDR1 to 3 set forth in SEQ ID NOs: 54 to 56, respectively); and 0211 antibody (having a heavy chain variable region having heavy chain CDR1 to 3 set forth in SEQ ID NOs: 57 to 59, respectively and a light chain variable region having light chain CDR1 to 3 set forth in SEQ ID NOs: 60 to 62, respectively), as the corresponding CDRs.

Examples of the antibody that binds to insoluble fibrin with a stronger affinity than to fibrinogen or an antigen-binding fragment thereof, include an antibody or an antigen-binding fragment thereof, containing:
a heavy chain variable region containing heavy chain CDR1 having the amino acid sequence set forth in SEQ ID NO: 10, heavy chain CDR2 having the amino acid sequence set forth in SEQ ID NO: 11 and heavy chain CDR3 having the amino acid sequence set forth in SEQ ID NO: 12; and
a light chain variable region containing light chain CDR1 having the amino acid sequence set forth in SEQ ID NO: 13, light chain CDR2 having the amino acid sequence set forth in SEQ ID NO: 14 and light chain CDR3 having the amino acid sequence set forth in SEQ ID NO: 15. The antibody may be a human chimeric antibody or a humanized antibody. According to the present invention, there is provided a humanized antibody having the above heavy chain variable region and light chain variable region.

Examples of the antibody that binds to insoluble fibrin with a stronger affinity than to fibrinogen or an antigen-binding fragment thereof, include an antibody or an antigen-binding fragment thereof, containing:
a heavy chain variable region having the amino acid sequence set forth in SEQ ID NO: 16; and
a light chain variable region having the amino acid sequence set forth in SEQ ID NO: 17. The antibody may further contain a heavy chain constant region in the Fab fragment or a part thereof in addition to a heavy chain variable region. The antibody may be a human chimeric antibody or a humanized antibody. According to the present invention, there is provided a humanized antibody having the above heavy chain variable region and light chain variable region.

Another example of the antibody that binds to insoluble fibrin with a stronger affinity than to fibrinogen or an antigen-binding fragment thereof is an antibody or an antigen-binding fragment thereof containing
a heavy chain variable region containing heavy chain CDR1 having the amino acid sequence set forth in SEQ ID NO: 18, heavy chain CDR2 having the amino acid sequence set forth in SEQ ID NO: 19, and heavy chain CDR3 having the amino acid sequence set forth in SEQ ID NO: 20, and
a light chain variable region containing light chain CDR1 having the amino acid sequence set forth in SEQ ID NO: 21, light chain CDR2 having the amino acid sequence set forth in SEQ ID NO: 22, and light chain CDR3 having the amino acid sequence set forth in SEQ ID NO: 23. The antibody can be a human chimeric antibody or a humanized antibody. According to the present invention, there is provided a humanized antibody having a heavy chain variable region and a light chain variable region as mentioned above.

The antibody that binds to insoluble fibrin with a stronger affinity than to fibrinogen or an antigen-binding fragment thereof is, for example, an antibody or an antigen-binding fragment thereof containing
a heavy chain variable region having the amino acid sequence set forth in SEQ ID NO: 24 and
a light chain variable region having the amino acid sequence set forth in SEQ ID NO: 25. The antibody may further contain a heavy chain constant region within a Fab fragment or a part thereof in addition to a heavy chain variable region. The antibody is a human chimeric antibody or a humanized antibody. According to the present invention, there is provided a humanized antibody having a heavy chain variable region and a light chain variable region as mentioned above.

According to the present invention, there may be provided an antibody competitive with the antibody described in the above (3) for binding to insoluble fibrin, preferably, a mutually competitive antibody. The antibody herein is preferably a humanized antibody.

According to the present invention, there is provided an antibody competitive with the antibody described in the above (4) for binding to insoluble fibrin, preferably, a mutually competitive antibody. The antibody herein is preferably a humanized antibody.

According to the present invention, the antibody described in the above (1) is a humanized antibody and can be an insoluble fibrin-specific antibody. According to the present invention, the antibody described in the above (2) is a humanized antibody and can be an insoluble fibrin-specific antibody. According to the present invention, the antibody described in the above (3) is a humanized antibody and can be an insoluble fibrin-specific antibody. According to the present invention, the antibody described in the above (4) is a humanized antibody and can be an insoluble fibrin-specific antibody. According to the present invention, the antibody competitive with the antibody described in the above (3) for binding to insoluble fibrin, preferably, a mutually competitive antibody, is a humanized antibody and can be an insoluble fibrin-specific antibody. According to the present invention, the antibody competitive with the antibody described in the above (4) for binding to insoluble fibrin, preferably, a mutually competitive antibody, is a humanized antibody and can be an insoluble fibrin-specific antibody.

The antibody and an antigen-binding fragment portion thereof according to the present invention can bind to a peptide consisting of the amino acid sequence set forth in SEQ ID NO: 8. The antibody of the present invention herein can be a humanized antibody.

In an embodiment, the fusion-protein variant of the present invention has the amino acid sequence set forth in SEQ ID NO: 1. In an embodiment, the fusion-protein variant of the present invention has the amino acid sequence set forth in SEQ ID NO: 2. In an embodiment, the fusion-protein variant of the present invention has the amino acid sequence set forth in SEQ ID NO: 3. In an embodiment, the fusion-protein variant of the present invention has the amino acid sequence set forth in SEQ ID NO: 26. In an embodiment, the fusion-protein variant of the present invention, the fusion-protein variant of the present invention has the amino acid sequence set forth in SEQ ID NO: 4. In an embodiment, the fusion-protein variant of the present invention, the fusion-protein variant of the present invention has the amino acid sequence set forth in SEQ ID NO: 5. In an embodiment, the fusion-protein variant of the present invention, the fusion-protein variant of the present invention has the amino acid sequence set forth in SEQ ID NO: 63.

### <Properties of variant>

The fusion-protein variant of the present invention may have at least one, preferably 2 or more, more preferably 3 or more, and further preferably all properties selected from the group consisting of (i) binding specificity to insoluble fibrin; (ii) binding affinity to insoluble fibrin; (iii) resistance against inhibition by a plasminogen activator inhibitor (PAI-1), and (iv) low ability to induce matrix metalloproteinase (MMP) activity in the tissue around thrombus. Because of this, it is possible to provide a safer and more effective therapy than conventional therapy using tissue plasminogen activator (tPA). Furthermore, it is possible to provide a new innovative technique compared to thrombolytic therapy with urokinase. In particular, it is more advantageous to production than the fusion protein disclosed in WO2021/200922A; at the same time, an effect of reducing side effects can be exerted.

### <Method for producing fusion-protein variant of the present invention>

According to the present invention, there is provided a method for producing a fusion-protein variant of the present invention. The fusion-protein variant of the present invention can be produced from protein-producing cells. For example, the variant can be produced from a protein-producing cell by temporally or preferably stably introducing a gene encoding the variant into the protein-producing cell and culturing the protein-producing cell in conditions suitable for producing the above variant.

Thus, according to the present invention, there is provided a protein-producing cell expressibly having a gene encoding a fusion-protein variant of the present invention. The protein-producing cell may be contained in a cell-freezing protection liquid or may be present in a frozen state. Thus, according to the present invention, there is provided a freeze-stock, a working cell bank, a master cell bank, and a research cell bank containing a protein-producing cell expressibly having a gene encoding a fusion-protein variant of the present invention. The fusion-protein variant of the present invention can be operably linked particularly to a control sequence. Because of this, the fusion-protein variant of the present invention can be expressed in a protein-producing cell.

According to the present invention, the fusion protein of the present invention can be prepared by a method known to those skilled in the art. For example, the fusion protein of the present invention can be expressed in cells (insect cells, bird cells, *Escherichia coli* cells, yeast cells, and mammalian cells), preferably, mammalian cells (for example, cells suitable for expressing a protein, for example, Chinese hamster ovary cells (CHO cells) and mammalian cells suitable for expressing a protein, such as 293 cells and cells derived from these like human cells). Examples of the protein-producing cell that can be used include CHO cells (for example, ExpiCHO^{™} cells, ExpiCHO-S^{™} cells, Free Style^{™} CHO cells). Expression can be made, for example, by using an expression vector, which contains a nucleic acid encoding a fusion protein of the present invention and is operatively linked to a promoter operational in the expression cells mentioned above. If a light chain is required for the fusion protein, the light chain can be co-expressed in the expression cells.

The fusion-protein variant of the present invention can be a solution to the problem of a low expression level of the fusion protein in a protein-producing cell. This is because the production of the fusion-protein variant of the present invention is increased by increasing culture time, although the production of a conventional fusion protein is not improved even if culture time is increased but the production of fragments increases. In addition, the activity of the fusion-protein variant of the present invention to degrade plasminogen in the presence of plasmin can be the same as in the conventional fusion protein.

In an embodiment, there is provided a fusion-protein variant excellent in stability and a method for producing a pharmaceutical composition containing the fusion-protein variant, comprising introducing an F157E or F157D mutation into the fusion protein of the present invention, thereby imparting excellent stability compared to the fusion protein not modified. In an embodiment, there is provided a method for improving the stability of a fusion-protein variant, comprising introducing an F157E or F157D mutation into the fusion protein of the present invention, thereby imparting more excellent stability to a fusion protein not modified.

Examples of the culture medium that can be used include, a serum-containing medium, a low serum medium (for example, serum concentration is less than 5%), a serum-free medium, a xeno-free medium and a chemically defined medium. In consideration of contamination with impurities, a serum-free medium, a xeno-free medium, or a chemically defined medium can be preferably used. The culture medium can be appropriately exchanged during culture. Furthermore, in order to reduce production cost, a culture medium without serum such as a serum-free medium, a xeno-free medium or a chemically defined medium can be preferably used. The fusion-protein variant of the present invention (containing an F157 variation) is advantageous over a non-fusion-protein variant in production since the fragmentation is suppressed in the absence of serum.

If a signal peptide is attached to a fusion protein, the fusion protein can be produced outside of a cell. In this case, the cell culture supernatant is collected and the fusion protein can be recovered from the supernatant. For purification of the fusion protein, it is possible to use various purification techniques known to those skilled in the art, such as gel filtration and affinity purification. Since the fusion-protein variant of the present invention (having a variation of F157) is resistant to fragmentation during purification, it is possible to easily control conditions including a temperature condition and a storage condition during purification. For example, it is necessary to purify a conventional fusion protein in the presence of L-arginine but the fusion-protein variant can be purified in the absence of L-arginine. Furthermore, since the fusion-protein variant of the present invention has high storage stability, the composition of the formulation can be set flexibly. In particular, L-arginine is not necessarily contained in the formulation.

The fusion-protein variant obtained is immobilized on the bottom surface of a plate, activated with plasmin before plasmin was washed away. The plasminogen degradation activity can be evaluated. For example, when a mutant activated and immobilized onto a plate is allowed to react with a substrate such as H-D-valyl-L-leucyl-L-lysyl-p-nitroanilide dihydrochloride in the presence of plasminogen, and plasminogen degradation activity of the fusion-protein variant can be determined by performing a colorimetric assay using p-nitroaniline, which exhibits color change upon substrate degradation.

### <Composition and pharmaceutical formulation>

According to the present invention, it is possible to provide a composition or a pharmaceutical formulation containing a fusion-protein variant of the present invention. The composition and pharmaceutical formulation may contain pharmaceutically acceptable additives in addition to the fusion-protein variant of the present invention. Examples of the additives include a pH buffer, an isotonic agent, a salt, a preservative and an analgesic. The pharmaceutical formulation can be prepared for intravenous administration or intra-arterial administration. In an embodiment, the pharmaceutical formulation of the present invention may be a lyophilized pharmaceutical formulation. In an embodiment, the present invention provides a kit (more specifically, a kit for preparing a formulation immediately before use) containing a lyophilized pharmaceutical formulation and water for injection.

The pharmaceutical formulation can be used for dissolving thrombi formed *in vivo.* Thus, the pharmaceutical formulation can be used for treating thrombosis. The pharmaceutical formulation can be further used for treating conditions or diseases caused by thrombosis. The pharmaceutical formulation can be also used for treating conditions or diseases to be suitably treated by dissolving fibrin clots. The pharmaceutical formulation can be also used for treating a thrombogenesis-associated cerebrovascular disorder and cardiovascular disorder. The pharmaceutical formulation can treat thrombogenesis-associated conditions or symptoms, for example, in a cerebrovascular disorder and a cardiovascular disorder, or whereby the conditions or symptoms of the cerebrovascular disorder and cardiovascular disorder can be treated. The treatment includes a therapeutic treatment and a prophylactic treatment. The therapeutic treatment includes reducing the rate and degree of worsening conditions or symptoms, stopping worsening them or improving the conditions or symptoms, whereas the prophylactic treatment includes preventing the development of conditions or symptoms or reducing incidence rates. Examples of the cerebrovascular disorder include an ischemic cerebrovascular disorder. Examples of the ischemic cerebrovascular disorder include cerebral infarction and transient ischemic attack. Cerebral infarction may be caused by cerebral thrombosis or cerebral embolism. Cerebral thrombosis is a condition where thrombi block the cerebral blood vessel, whereas cerebral embolism is a condition where thrombi formed in the vessel except the brain are transported to the brain and block the blood vessel. Examples of the cardiovascular disorder include ischemic heart disease. Examples of the ischemic heart disease include angina pectoris and myocardial infarction. The pharmaceutical formulation may be preferably used in the acute phases of these diseases. This is because tissue damages caused by formation of thrombi and emboli can be prevented if the pharmaceutical formulation is used in the acute phases. Examples of the acute phase may include acute phases within one hour, within 2 hours, within 3 hours, within 4 hours, within 4.5 hours, within 5 hours, within 6 hours, within 12 hours, within or 24 hours after the onset of a disease. For example, for an ischemic cerebrovascular disorder, it is recommended to administer a thrombolytic agent within 4.5 hours from the onset thereof, whereas for an ischemic cardiovascular disorder, it is recommended to administer a thrombolytic agent within 6 hours from the onset thereof. The pharmaceutical formulation of the present invention can be administered in the same manner.

According to the present invention, the fusion-protein variant of the present invention can dissolve fibrin clots. In an embodiment, the fibrin clots are thrombi.

### <Imaging agent>

According to the present invention, the fusion-protein variant of the present invention may be linked to an imaging agent (a covalent bond is contained). The variant and the imaging agent may be linked via a linker or directly linked without a linker. The variant linked to an imaging agent can be used in visualizing *in vivo* fibrin clots (particularly thrombi) as images and thus used for observing thrombi in a body. As the imaging agent, a contrast agent can be used. Examples of the contrast agent may include contrast agents for use in, e.g., magnetic resonance imaging (MRI), computed tomography (CT), and positron emission tomography (PET). Thus, according to the present invention, there is provided a formulation or composition containing a fusion-protein variant of the present invention linked to an imaging agent, for use in in-vivo imaging.

According to the present invention, there is provided a method for treating thrombosis or a method for dissolving fibrin clots in a subject who needs it, comprising administering a therapeutically effective amount of a fusion-protein variant of the present invention to the subject.

According to the present invention, there is provided a pharmaceutical formulation comprising a therapeutically effective amount of a fusion-protein variant of the present invention for use in a method for treating thrombosis or a method for dissolving fibrin clots in a subject who needs it.

According to the present invention, there is provided a fusion-protein variant of the present invention for use in a method for treating thrombosis or a method for dissolving fibrin clots in a subject who needs it.

According to the present invention, there is provided use of a fusion-protein variant of the present invention in producing a medicament for use in a method for treating thrombosis or a method for dissolving fibrin clots in a subject who needs it.

According to the present invention, there is provided a method for treating conditions or symptoms of a cerebrovascular disorder and a cardiovascular disorder in a subject who needs it, comprising administering a therapeutically effective amount of the fusion-protein variant of the present invention to the subject.

According to the present invention, there is provided a pharmaceutical formulation comprising the fusion-protein variant of the present invention for use in the method for treating conditions or symptoms of a cerebrovascular disorder and a cardiovascular disorder in a subject who needs it.

According to the present invention, there is provided a fusion-protein variant of the present invention for use in the method for treating conditions or symptoms of a cerebrovascular disorder and a cardiovascular disorder in a subject who needs it.

There is provided use of a fusion-protein variant of the present invention in producing a medicament for use in treating conditions or symptoms of a cerebrovascular disorder and a cardiovascular disorder in a subject who needs it.

### Examples

### Example 1: Preparation of Fusion Protein Dissolving Fibrin Clots

In WO2021/200922A, a fusion protein (hereinafter, referred to as "AMU1114"), which contains an antibody fragment showing specific binding to insoluble fibrin and a prourokinase mutant, was synthesized. AMU1114 is a complex of a fusion protein (heavy chain), which is formed by linking a Fab region of an antibody to a prourokinase mutant, and a light chain of an antibody.

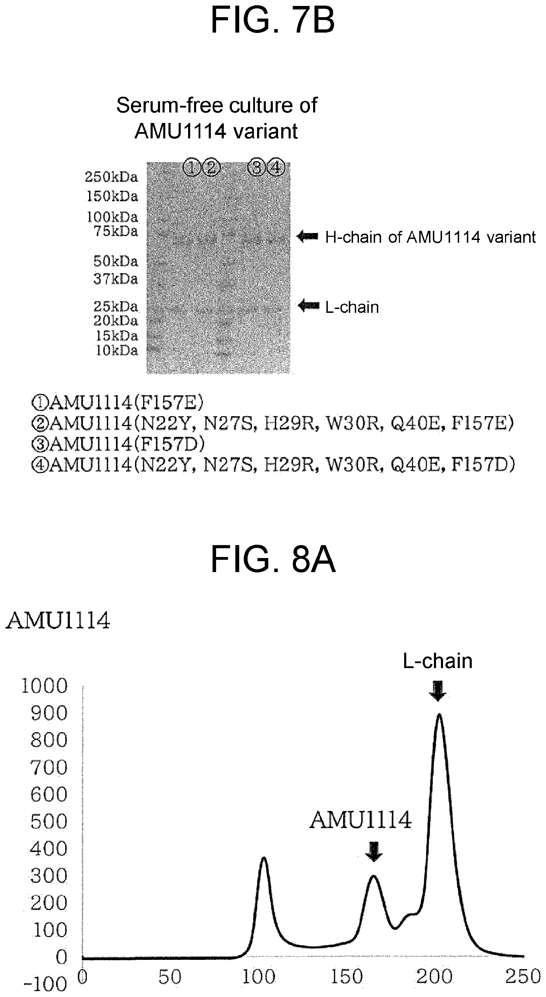

When AMU1114 was expressed in Chinese hamster ovary cells (CHO cells), the expression level reached a plateau Day 5 to 7 after initiation of culture. Even if culture was subsequently continued, the expression level of AMU1114 did not increase. More specifically, as shown in Figure 8A, the production of AMU1114 did not increase and the recovered amount of the light chain (L chain) only increased.

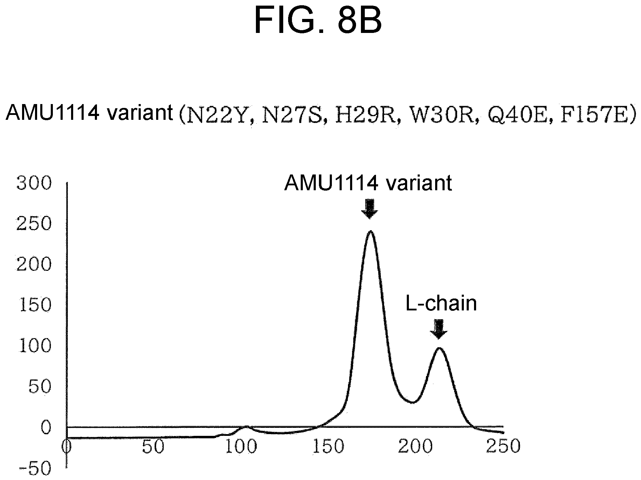

Culture conditions were examined. When AMU1114 was expressed in the CHO cells in a serum-free medium, the yield of the light chain increased but the yield of AMU1114 decreased, as shown in Figures 8A and 8B. However, when AMU1114 was expressed in the CHO cells in the presence of 20% fetal bovine serum (FBS), the yield of AMU1114 increased but the yield of the light chain decreased. Furthermore, for the purification of AMU1114 in the CHO cells, the presence of L-arginine was essential. The recovered amount of AMU1114 purified was 25 to 35 mg/L.

### Example 2: Preparation of Fusion-protein variant

In this Example, variants of AMU1114 were prepared. More specifically, a mutation was introduced into a site of F396 (corresponding to F157 of urokinase) of an AMU1114 heavy chain by amino acid substitution in a site-specific manner. The mutation was introduced by amino acid substitution in accordance with a PCR method using DNA encoding AMU1114 as a template and primers for introducing a mutation. As the PCR enzyme, PrimeStar HS DNA polymerase (Takara) was used.

XhoI (Takara) and NotI (Takara) were allowed to react with AMU1114 incorporated into pcDNA3.4 (ThermoFisher) and each of the PCR products in accordance with the manufacturer's protocol for 2 hours, and subsequently, 1% agarose gel electrophoresis was performed. Thereafter, purification was performed by FastGene Gel/PCR Extraction Kit (Nippon Genetics). A competent High DH5α (Toyobo) cell was transformed and cultured in an LB medium (50 µg/mL) plate for 16 hours. From the cell (*Escherichia coli*) transformed, a plasmid was extracted using FastGene Plasmid Mini Kit (Nippon Genetics) and sequenced. By the sequencing, it was confirmed that the mutation was introduced as desired.

AMU1114 and mutants were expressed in the CHO cells. More specifically, AMU1114 and mutants were temporarily expressed using the ExpiCHO Expression System (Thermo Fisher). After culture was performed for 7 days, the culture supernatant was collected. To the culture medium, FBS was added so as to have a concentration of 20%. The culture supernatant containing products temporally expressed was added to CaptoL (Cytiva) equilibrated with PBS, washed with the same buffer, and eluted with a 100 mM glycine buffer, pH 3.0, to obtain a final product.

The concentration of each mutant was adjusted with PBS to 10 µg/mL and the resultant solution was dispensed in the wells of a 96-well plate in a volume of 100 µL/well, allowed to stand still at room temperature for 90 minutes to immobilize the mutant. To the wells of the plate having the mutant immobilized thereon, TBS-T containing 1% BSA was dispensed in a volume of 300 µL/well. The plate was allowed to stand still at room temperature for 60 minutes to block the plate. After the plate blocked was washed with TBS-T, a reaction solution containing 5 µg/mL plasminogen and a 15 µL/well substrate (Test Team S PLG Sekisui Medical Co., Ltd.) and adjusted with PBS, was added in a volume of 150 µL/well, and absorbances at 405 nm and 505 nm were measured every 5 minutes for 2 hours.

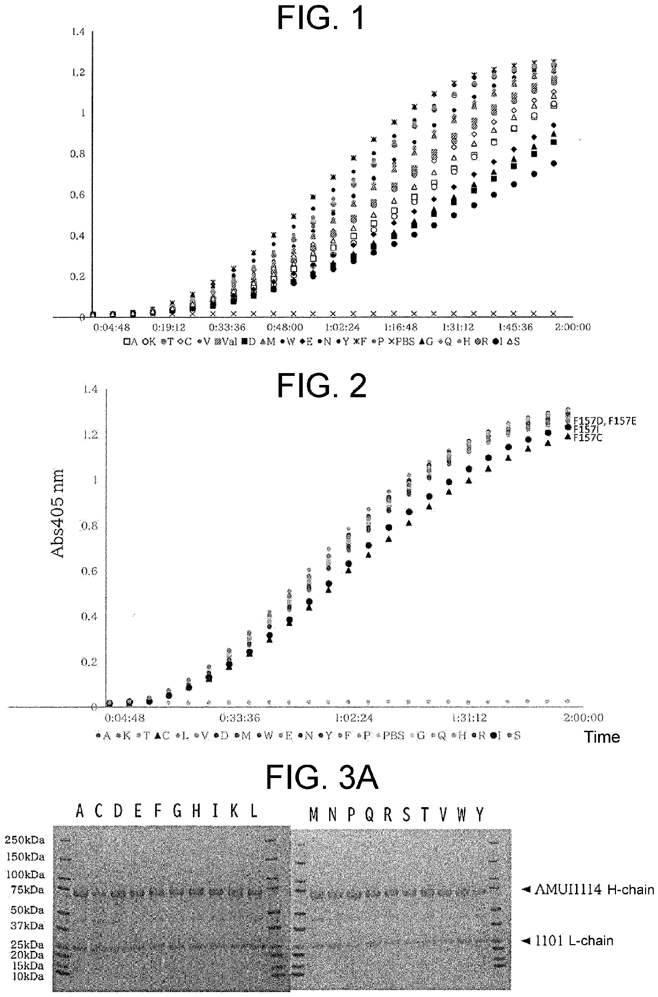

The results were as shown in Figure 1. As shown in Figure 1, it was found that the reactivity to a substrate decreased in all mutants except F157Y mutant; the reactivity to a substrate greatly decreased in mutants (F157A, F157K, F157C, and F157S); and the reactivity to a substrate more greatly decreased in mutants (F157I, F157G, F157D, and F157E). This is considered because these mutants have resistance to plasmin.

The concentration of each variant was adjusted to 10 µg/mL with PBS. The resultant solution was dispensed in the wells of a 96-well plate in a volume of 100 µL/well and allowed to stand still at room temperature for 90 minutes to immobilize the variant. To the plate having the variant immobilized thereon, TBS-T containing 1% BSA was dispensed in a volume of 300 µL/well. The plate was allowed to stand still at room temperature for 60 minutes to block the plate. To the plate blocked, 5 µg/mL plasmin solution was added in a volume of 100 µl/well and allowed to react at 37°C for 60 minutes.

After washing with TBS-T, a reaction solution containing 5 µg/mL plasminogen and a 15 µL/well substrate (Test Team S PLG Sekisui Medical Co., Ltd.) and adjusted with PBS, was added in a volume of 150 µL/well, and absorbances at 405 nm and 505 nm were measured every 5 minutes for 2 hours.

The results were as shown in Figure 2. As shown in Figure 2, all mutants treated with plasmin exhibited high reactivity to the substrate. Particularly, the activities of F157E and F157D mutants were equivalent to that of AMU1114.

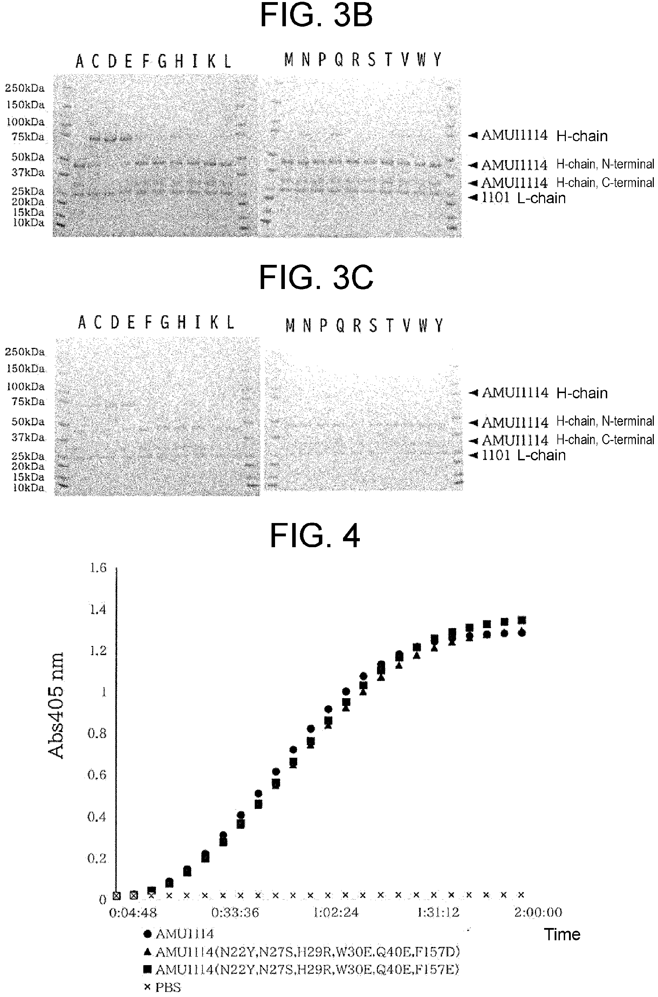

The concentration of each purified F157 variant was adjusted with PBS to 1 mg/mL. The variant was allowed to stand still at 37°C, sampled at the time of 0 (Figure 3A), after 24 hours (Figure 3B) and after 120 hours (Figure 3C). The samples were checked by SDS-PAGE electrophoresis. In Figures 3A to 3C, the mutants are represented by single letters representing amino acids substituted for the F157 position. it was found that the F157D mutant and F157E mutant particularly have high stability. Furthermore, the yields of F157E mutant and F157D mutant were 3 to 5 times as high as that of AMU1114.

### Example 3: Binding Ability to Urokinase Receptor

In this Example, the binding affinity of AMU1114 to a human urokinase receptor was checked. Since the binding affinity was confirmed, mutations of N22Y, N27S, H29R, W30R and Q40E were further introduced into each of F157D mutant and F157E mutant (see, Miyake T, et al., J Biochem. 1988; 104 (4): 643-647, Magdolen V, et al., Eur J Biochem. 1996; 237 (3): 743-751, and Lin L, et al., J Biol Chem. 2010; 285 (14): 10982-92).

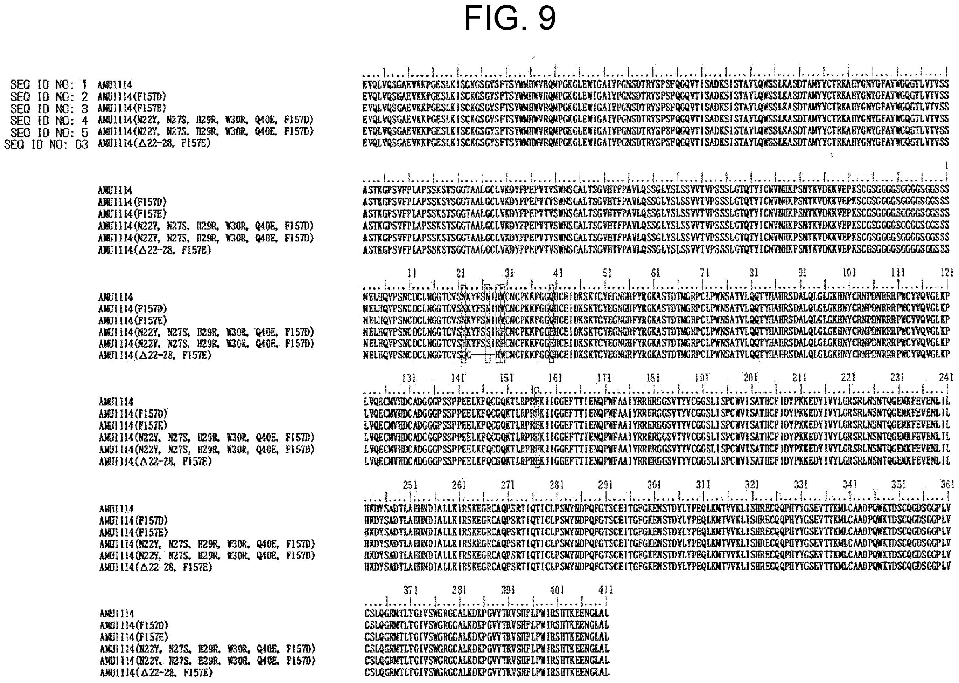

An AMU1114 mutant (N22G, K23G, Y24_I28del, F157E) was prepared by site-specific mutagenesis. Hereinafter, the mutant will be referred to as an AMU1114 (Δ22-28, F157E) mutant. The AMU1114 (Δ22-28, F157E) mutant has the amino acid sequence set forth in SEQ ID NO: 63. The multiple alignments of the amino acid sequence of the mutant prepared are shown in Figure 9. The yield was calculated as 100 mg/L.

As the urokinase receptor, a human urokinase mutant having the amino acid sequence set forth in SEQ ID NO: 6 and a mouse urokinase receptor having the amino acid sequence set forth in SEQ ID NO: 7 were used. To the C terminal of each urokinase, 6 × His tag was attached and affinity purification was performed.

More specifically, EcoRI (Takara) and XhoI (Takara) were allowed to react with pcDNA3.3 (ThermFisher) and PCR products of the urokinase receptors in accordance with the manufacturer's protocol for 2 hours and 1% agarose gel electrophoresis was performed. Thereafter, purification was performed by use of FastGene Gel/PCR Extraction Kit (Nippon Genetics). Using Ligation High (takara), the vector (pcDNA3.3) and an insert were reacted for 30 minutes. Competent High DH5α (toyobo) cell was transformed and cultured in an LB medium (50 µg/mL) plate for 16 hours. From the cell (*Escherichia coli*) transformed, a plasmid was extracted by use of FastGene Plasmid Mini Kit (Nippon Genetics) and sequenced. Using the ExpiCHO Expression System (Thermo Fisher), a urokinase mutant was temporarily expressed. The culture was performed for 12 days and the culture supernatant was collected. The culture supernatant containing products temporally expressed was dialyzed against 50 mM Tris-HCl, pH 8.5, 300 mM NaCl, added in Ni column (Cytiva) equilibrated with the same buffer, washed with the same buffer and eluted with PBS containing 400 mM imidazole to obtain a final product.

Variants were purified. An AMU1114 (N22Y, N27S, H29R, W30R, Q40E, F157D) mutant, an AMU1114 (N22Y, N27S, H29R, W30R, Q40E, F157E) mutant, and an AMU1114 (Δ22-28, F157E) mutant were temporally expressed in a serum-free medium using the ExpiCHO Expression System (Thermo Fisher) alone. Culture was performed for 12 days and the culture supernatant was collected. The culture supernatant containing products temporally expressed was added in CaptoL (Cytiva) equilibrated with PBS, washed with the same buffer and eluted with 100 mM glycine buffer, pH 3.0. The sample eluted was subjected to purification by gel filtration using Superdex200pg (Cytiva) to obtain a final product.

The concentration of each variant was adjusted with PBS to 10 µg/mL and the resultant solution was dispensed in the wells of a 96-well plate in a volume of 100 µL/well, allowed to stand still at room temperature for 90 minutes to immobilize the variant. To the plate having the variant immobilized thereon, TBS-T containing 1% BSA was dispensed in a volume of 300 µL/well. The plate was allowed to stand still at room temperature for 60 minutes to block the plate. To the plate blocked, a plasmin solution adjusted in concentration to 5 µg/mL was added in a volume of 100 µL/well and allowed to react at 37°C for 60 minutes. After washing with TBS-T, a reaction solution containing 5 µg/mL plasminogen and a 15 µL/well substrate (Test Team S PLG Sekisui Medical Co., Ltd.) and adjusted with PBS, was added in a volume of 150 µL/well, and absorbances at 405 nm and 505 nm were measured every 5 minutes for 2 hours.

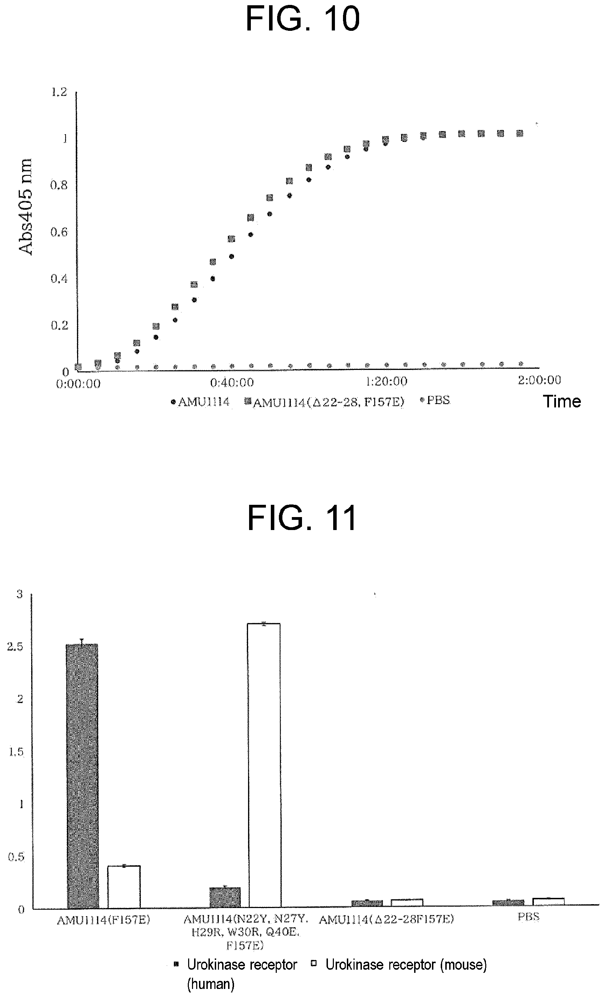

The results were as shown in Figures 4 and 10. As shown in Figures 4 and 10, 3 mutants all exhibited the same reactivity as AMU1114 in the presence of plasmin.

The concentration of each of the human and mouse urokinase receptors was adjusted with PBS to 1 µg/mL. The resultant solution was dispensed in the wells of a 96-well plate in a volume of 50 µL/well, allowed to stand still at room temperature for 90 minutes to immobilize the receptor. To the plate having the receptor immobilized thereon, TBS-T containing 1% BSA was dispensed in a volume of 300 µL/well. The plate was allowed to stand still at room temperature for 60 minutes to block the plate. To the plate blocked, the sample (mutant) adjusted in concentration to 1000 ng/mL, 111.1 ng/mL or 12.3 ng/mL was added in a volume of 50 µL/well and allowed to react at room temperature for 60 minutes. After washing with TBS-T, HRP-conjugated anti-human Kappa chain antibody (Bethyl) diluted with 1% BSA-containing TBS-T to 1/50,000 fold was dispensed in a volume of 50 µL/well and allowed to react at room temperature for 60 minutes. After washing, a TMB (3,3',5,5'-tetramethylbenzidine) coloring reagent was allowed to react for 15 minutes. The reaction was terminated with 2N H₂SO₄ (30 µL/well). Absorbance at 450 nm was measured.

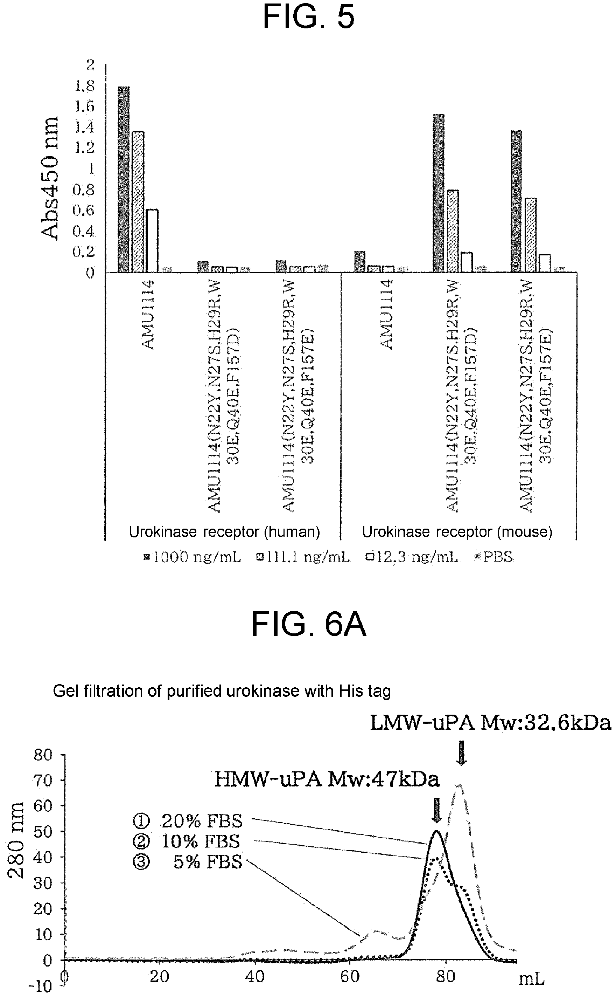

The results were as shown in Figure 5. As shown in Figure 5, it was found, for the first time, that AMU1114 has a binding affinity to a human urokinase receptor. Then, the binding affinity of the above 3 mutants to a human urokinase receptor was examined. As shown in Figures 5 and 11, 3 mutants lost the binding affinity to a human urokinase mutant. Furthermore, as shown in Figure 11, the AMU1114 (Δ22-28, F157E) mutant did not exhibit significant binding to the human urokinase receptor or the mouse urokinase receptor, as is apparent from comparison with a negative control, PBS. Further, an AMU1114 mutant, which was prepared so as to have F157E/D and W30R, also had an extremely lower binding affinity to the human urokinase receptor.

Urokinase prepared by adding His-tag to C terminal thereof was expressed in ExpiCHOs containing 5%, 10% and 20% FBS, respectively. After the culture was performed for 12 days, the culture supernatant was dialyzed against 50 mM Tris-HCl pH 8.5, 300 mM NaCl, added in Ni column (Cytiva) equilibrated with the same buffer, washed with the same buffer and eluted with PBS containing 400 mM imidazole. The sample eluted was subjected to gel filtration using Superdex75pg (Cytiva) to obtain a final sample.

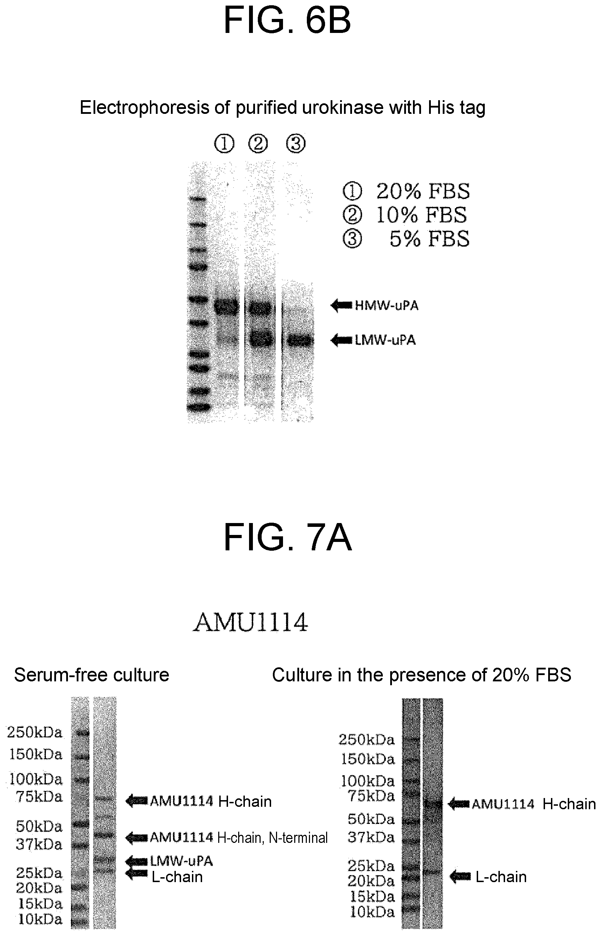

The results were as shown in Figures 6A and 6B. As is shown in Figures 6A and 6B, when cell culture was carried out in the presence of 5% FBS, a low-molecular weight uPA was observed. When cell culture was carried out in the presence of 20% FBS, the amount of the low-molecular weight uPA decreased. Accordingly, it was demonstrated that it is necessary to perform cell culture in the presence of FBS for expressing uPA.

AMU1114 was similarly expressed in ExpiCHO in the presence of 20% FBS or a serum-free condition. When AMU1114 was purified and electrophoresed as mentioned above, the presence of various fragments was observed in the serum-free condition (see, Figure 7A) but the amount of fragments significantly decreased in the presence of a 20% FBS (see, Figure 7A).

Subsequently, 4 types of AMU1114 variants were similarly expressed in ExpiCHO in a serum-free condition. When the variants were purified and electrophoresed as mentioned above, these mutants were not fragmented even if they were cultured in a serum-free condition (see, Figure 7B).

AMU1114 and AMU1114 variants were subjected to affinity purification and thereafter analyzed by gel filtration. The results were as shown in Figures 8A and 8B. As shown in Figures 8A and 8B, in AMU1114, it was observed that the peak of a light chain is higher than AMU1114. In contrast, in the AMU1114 variant, the peak of a light chain was kept at a low level and proportionally the peak of the AMU1114 variant appeared to be high.

From the results, it was found that an F157 mutant of AMU1114 exhibits the same physiological activity as AMU1114 in the presence of plasmin but has higher stability than AMU1114 and is less contaminated with free light-chains. Thus, the mutant has a high purity and further the production thereof was 3 to 5 times as high as that of AMU1114.

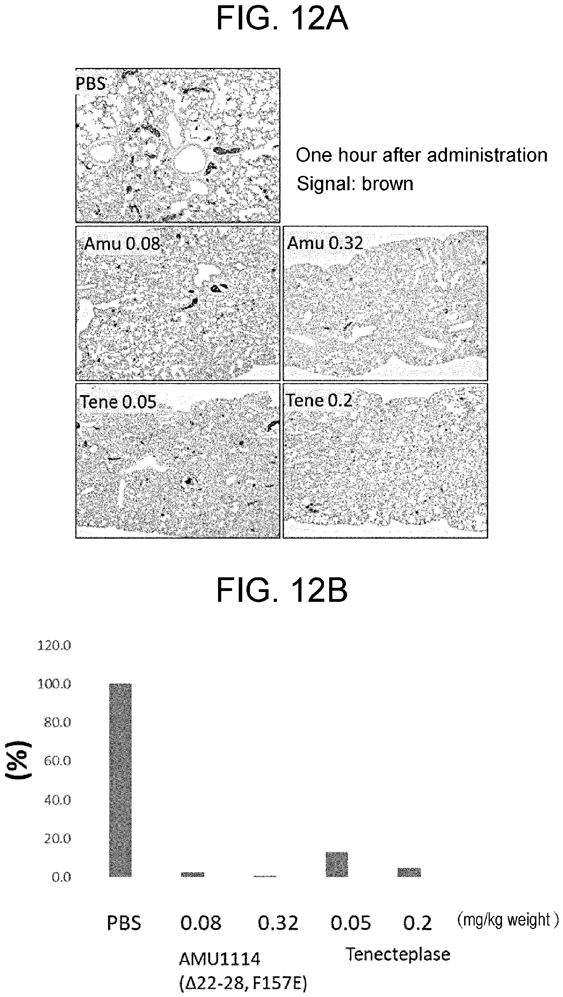

Thrombin thrombosis model mice were administered with AMU1114 (Δ22-28, F157E) and compared for the degree of thrombolysis with a tenecteplase administration group, and a negative control group. To describe more specifically, thrombin (600 U/kg) was intravenously injected to Balb/c female mice to prepare thrombin thrombosis model mice. Thirty minutes after the intravenous injection, PBS (negative control), tenecteplase (positive control) and AMU1114 (Δ22-28, F157E) were intravenously administered. Further, 60 minutes later, the lung was excised from each of the mice under deep anesthesia and thrombi were stained directly with a labeled anti-insoluble fibrin antibody (1101 antibody). The total area of signals (brown) corresponding to thrombi was calculated. The higher the degree of thrombolysis, the lower the total area. As a result, as shown in Figure 12A and Figure 12B and Table 1, it was found that a huge number of thrombi was observed in the negative control group but thrombi were rarely observed in the positive control group and AMU1114 (Δ22-28, F157E) administered group.

### [Table 1]

**Table 1: Test Results of Degree of Thrombolysis**

| | **NT** | **AMU1114 (Δ22-28, F157E)** | | **Tenecteplase** | |
|---|---|---|---|---|---|
| Dosage mg/kg | - | 0.08 | 0.32 | 0.05 | 0.2 |
| Total area of signals | 59524 | 1483 | 480 | 7837 | 2731 |
| % | 100.0 | 2.5 | 0.8 | 13.2 | 4.6 |

### Content of Sequence Listing

SEQ ID NO: 1: Amino acid sequence of AMU1114 heavy chain 
EVQLVQSGAEVKKPGESLKISCKGSGYSFTSYWMHWVRQMPGKGLEWIGAIYPGNSD
TRYSPSFQGQVTISADKSISTAYLQWSSLKASDTAMYYCTRKAHYGNYGFAYWGQGT
LVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCGSGGG
GSGGGGSGGSSSNELHQVPSNCDCLNGGTCVSNKYFSNIHWCNCPKKFGGQHCEIDK
SKTCYEGNGHFYRGKASTDTMGRPCLPWNSATVLQQTYHAHRSDALQLGLGKHNYCR
NPDNRRRPWCYVQVGLKPLVQECMVHDCADGGGPSSPPEELKFQCGQKTLRPRFKII
GGEFTTIENQPWFAAIYRRHRGGSVTYVCGGSLISPCWVISATHCFIDYPKKEDYIV
YLGRSRLNSNTQGEMKFEVENLILHKDYSADTLAHHNDIALLKIRSKEGRCAQPSRT
IQTICLPSMYNDPQFGTSCEITGFGKENSTDYLYPEQLKMTVVKLISHRECQQPHYY
GSEVTTKMLCAADPQWKTDSCQGDSGGPLVCSLQGRMTLTGIVSWGRGCALKDKPGV
YTRVSHFLPWIRSHTKEENGLALSAGGAS
SEQ ID NO: 2: Amino acid sequence of AMU1114 F157D heavy chain 
EVQLVQSGAEVKKPGESLKISCKGSGYSFTSYWMHWVRQMPGKGLEWIGAIYPGNSD

TRYSPSFQGQVTISADKSISTAYLQWSSLKASDTAMYYCTRKAHYGNYGFAYWGQGT

LVTVSSASTKGPSVFPLAPSSKSTSGGTAALGCLVKDYFPEPVTVSWNSGALTSGVH

TFPAVLQSSGLYSLSSVVTVPSSSLGTQTYICNVNHKPSNTKVDKKVEPKSCGSGGG
 
GSGGGGSGGSSSNELHQVPSNCDCLNGGTCVSNKYFSNIHWCNCPKKFGGQHCEIDK

SKTCYEGNGHFYRGKASTDTMGRPCLPWNSATVLQQTYHAHRSDALQLGLGKHNYCR

NPDNRRRPWCYVQVGLKPLVQECMVHDCADGGGPSSPPEELKFQCGQKTLRPRDKII

GGEFTTIENQPWFAAIYRRHRGGSVTYVCGGSLISPCWVISATHCFIDYPKKEDYIV

YLGRSRLNSNTQGEMKFEVENLILHKDYSADTLAHHNDIALLKIRSKEGRCAQPSRT

IQTICLPSMYNDPQFGTSCEITGFGKENSTDYLYPEQLKMTVVKLISHRECQQPHYY

GSEVTTKMLCAADPQWKTDSCQGDSGGPLVCSLQGRMTLTGIVSWGRGCALKDKPGV

YTRVSHFLPWIRSHTKEENGLALSAGGAS
SEQ ID NO: 3: Amino acid sequence of AMU1114 F157E heavy chain 
EVQLVQSGAEVKKPGESLKISCKGSGYSFTSYWMHWVRQMPGKGLEWIGAIYPGNSD
TRYSPSFQGQVTISADKSISTAYLQWSSLKASDTAMYYCTRKAHYGNYGFAYWGQGT
LVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCGSGGG
GSGGGGSGGSSSNELHQVPSNCDCLNGGTCVSNKYFSNIHWCNCPKKFGGQHCEIDK
SKTCYEGNGHFYRGKASTDTMGRPCLPWNSATVLQQTYHAHRSDALQLGLGKHNYCR
NPDNRRRPWCYVQVGLKPLVQECMVHDCADGGGPSSPPEELKFQCGQKTLRPREKII
GGEFTTIENQPWFAAIYRRHRGGSVTYVCGGSLISPCWVISATHCFIDYPKKEDYIV
YLGRSRLNSNTQGEMKFEVENLILHKDYSADTLAHHNDIALLKIRSKEGRCAQPSRT
IQTICLPSMYNDPQFGTSCEITGFGKENSTDYLYPEQLKMTVVKLISHRECQQPHYY
GSEVTTKMLCAADPQWKTDSCQGDSGGPLVCSLQGRMTLTGIVSWGRGCALKDKPGV
YTRVSHFLPWIRSHTKEENGLALSAGGAS
SEQ ID NO: 4: Amino acid sequence of AMU1114 F157D, further modified heavy chain 
EVQLVQSGAEVKKPGESLKISCKGSGYSFTSYWMHWVRQMPGKGLEWIGAIYPGNSD

TRYSPSFQGQVTISADKSISTAYLQWSSLKASDTAMYYCTRKAHYGNYGFAYWGQGT

LVTVSSASTKGPSVFPLAPSSKSTSGGTAALGCLVKDYFPEPVTVSWNSGALTSGVH
 
TFPAVLQSSGLYSLSSVVTVPSSSLGTQTYICNVNHKPSNTKVDKKVEPKSCGSGGG

GSGGGGSGGSSSNELHQVPSNCDCLNGGTCVSYKYFSSIRRCNCPKKFGGEHCEIDK

SKTCYEGNGHFYRGKASTDTMGRPCLPWNSATVLQQTYHAHRSDALQLGLGKHNYCR

NPDNRRRPWCYVQVGLKPLVQECMVHDCADGGGPSSPPEELKFQCGQKTLRPRDKII

GGEFTTIENQPWFAAIYRRHRGGSVTYVCGGSLISPCWVISATHCFIDYPKKEDYIV

YLGRSRLNSNTQGEMKFEVENLILHKDYSADTLAHHNDIALLKIRSKEGRCAQPSRT

IQTICLPSMYNDPQFGTSCEITGFGKENSTDYLYPEQLKMTVVKLISHRECQQPHYY

GSEVTTKMLCAADPQWKTDSCQGDSGGPLVCSLQGRMTLTGIVSWGRGCALKDKPGV

YTRVSHFLPWIRSHTKEENGLALSAGGAS
SEQ ID NO: 5: Amino acid sequence of AMU1114 F157E, further modified 
EVQLVQSGAEVKKPGESLKISCKGSGYSFTSYWMHWVRQMPGKGLEWIGAIYPGNSD
TRYSPSFQGQVTISADKSISTAYLQWSSLKASDTAMYYCTRKAHYGNYGFAYWGQGT
LVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCGSGGG
GSGGGGSGGSSSNELHQVPSNCDCLNGGTCVSYKYFSSIRRCNCPKKFGGEHCEIDK
SKTCYEGNGHFYRGKASTDTMGRPCLPWNSATVLQQTYHAHRSDALQLGLGKHNYCR
NPDNRRRPWCYVQVGLKPLVQECMVHDCADGGGPSSPPEELKFQCGQKTLRPREKII
GGEFTTIENQPWFAAIYRRHRGGSVTYVCGGSLISPCWVISATHCFIDYPKKEDYIV
YLGRSRLNSNTQGEMKFEVENLILHKDYSADTLAHHNDIALLKIRSKEGRCAQPSRT
IQTICLPSMYNDPQFGTSCEITGFGKENSTDYLYPEQLKMTVVKLISHRECQQPHYY
GSEVTTKMLCAADPQWKTDSCQGDSGGPLVCSLQGRMTLTGIVSWGRGCALKDKPGV
YTRVSHFLPWIRSHTKEENGLALSAGGAS
SEQ ID NO: 6: Amino acid sequence of pro-urokinase 
SNELHQVPSNCDCLNGGTCVSNKYFSNIHWCNCPKKFGGQHCEIDKSKTCYEGNGHF

YRGKASTDTMGRPCLPWNSATVLQQTYHAHRSDALQLGLGKHNYCRNPDNRRRPWCY

VQVGLKPLVQECMVHDCADGKKPSSPPEELKFQCGQKTLRPRFKIIGGEFTTIENQP
 
WFAAIYRRHRGGSVTYVCGGSLISPCWVISATHCFIDYPKKEDYIVYLGRSRLNSNT

QGEMKFEVENLILHKDYSADTLAHHNDIALLKIRSKEGRCAQPSRTIQTICLPSMYN

DPQFGTSCEITGFGKENSTDYLYPEQLKMTVVKLISHRECQQPHYYGSEVTTKMLCA

ADPQWKTDSCQGDSGGPLVCSLQGRMTLTGIVSWGRGCALKDKPGVYTRVSHFLPWI

RSHTKEENGLAL
SEQ ID NO: 7: pro-urokinase K135G, Amino acid sequence of K136G 
SNELHQVPSNCDCLNGGTCVSNKYFSNIHWCNCPKKFGGQHCEIDKSKTCYEGNGHF
YRGKASTDTMGRPCLPWNSATVLQQTYHAHRSDALQLGLGKHNYCRNPDNRRRPWCY
VQVGLKPLVQECMVHDCADGGGPSSPPEELKFQCGQKTLRPRFKIIGGEFTTIENQP
WFAAIYRRHRGGSVTYVCGGSLISPCWVISATHCFIDYPKKEDYIVYLGRSRLNSNT
QGEMKFEVENLILHKDYSADTLAHHNDIALLKIRSKEGRCAQPSRTIQTICLPSMYN
DPQFGTSCEITGFGKENSTDYLYPEQLKMTVVKLISHRECQQPHYYGSEVTTKMLCA
ADPQWKTDSCQGDSGGPLVCSLQGRMTLTGIVSWGRGCALKDKPGVYTRVSHFLPWI
RSHTKEENGLAL
SEQ ID NO: 8: Amino acid sequence of Fibrinogen Bbeta 231-246 CNIPVVSGKECEEIIR
SEQ ID NO: 9: Amino acid sequence of Fibrinogen gamma 232-246 KNWIQYKEGFGHLSP
SEQ ID NO: 10: Amino acid sequence of Humanized 1101 HCDR1 SYWMH
SEQ ID NO: 11: Amino acid sequence of Humanized 1101 HCDR2 AIYPGNSDTRYSPSFQG
SEQ ID NO: 12: Amino acid sequence of Humanized 1101 HCDR3 KAHYGNYGFAY
SEQ ID NO: 13: Amino acid sequence of Humanized 1101 LCDR1 RASQHINNWLA
SEQ ID NO: 14: Amino acid sequence of Humanized 1101 LCDR2 GATSLQS
SEQ ID NO: 15: Amino acid sequence of Humanized 1101 LCDR3 QQYWSTPLT
SEQ ID NO: 16: Amino acid sequence of Humanized 1101 VH 
MGSTAILALLLAVLQGVCAEVQLVQSGAEVKKPGESLKISCKGSGYSFTSYWMHWVR

QMPGKGLEWIGAIYPGNSDTRYSPSFQGQVTISADKSISTAYLQWSSLKASDTAMYY

CTRKAHYGNYGFAYWGQGTLVTVSSASTKGPSVFPLAPSSKSTSGGTAALGCLVKDY

FPEPVTVSWNSGALTSGVHTFPAVLQSSGLYSLSSVVTVPSSSLGTQTYICNVNHKP

SNTKVDKKVEPKSC
SEQ ID NO: 17: Amino acid sequence of Humanized 1101 VL 
MDMRVPAQLLGLLLLWLRGARCDIQMTQSPSSLSASVGDRVTITCRASQHINNWLAW
YQQKPGKAPKLLISGATSLQSGVPSRFSGSGSGTDFTLTISSLQPEDFATYYCQQYW
STPLTFGQGTKVEIKRTVAAPSVFIFPPSDEQLKSGTASVVCLLNNFYPREAKVQWK
VDNALQSGNSQESVTEQDSKDSTYSLSSTLTLSKADYEKHKVYACEVTHQGLSSPVT
KSFNRGEC
SEQ ID NO: 18: Amino acid sequence of Humanized 99 HCDR1 NYGMN
SEQ ID NO: 19: Amino acid sequence of Humanized 99 HCDR2 WINTKIGEPTYAQKFQG
SEQ ID NO: 20: Amino acid sequence of Humanized 99 HCDR3 LLDY
SEQ ID NO: 21: Amino acid sequence of Humanized 99 LCDR1 RASQSVLYSSNQKNYLA
SEQ ID NO: 22: Amino acid sequence of Humanized 99 LCDR2 WASSLQS
SEQ ID NO: 23: Amino acid sequence of Humanized 99 LCDR3 HQYLSSYT
SEQ ID NO: 24: Amino acid sequence of Humanized 99 VH 
QVQLVQSGAEVKKPGASVKVSCKASGYTFTNYGMNWVRQAPGQGLEWMGWINTKIGE
PTYAQKFQGRVTMTRDKSSSTAYMELSSLRSEDTAVYYCARLLDYWGQGTLVTVSS
SEQ ID NO: 25: Amino acid sequence of Humanized 99 VL 
DIQMTQSPSSLSASVGDRVTITCRASQSVLYSSNQKNYLAWYQQKPGKSPKLLIYWA

SSLQSGVPSRFSGSGSGTDFTLTISSLQPEDFATYYCHQYLSSYTFGQGTKVEIKR
SEQ ID NO: 26: Amino acid sequence of AMU1114 F157E/D, further modified 
EVQLVQSGAEVKKPGESLKISCKGSGYSFTSYWMHWVRQMPGKGLEWIGAIYPGNSD
TRYSPSFQGQVTISADKSISTAYLQWSSLKASDTAMYYCTRKAHYGNYGFAYWGQGT
LVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCGSGGG
GSGGGGSGGSSSNELHQVPSNCDCLNGGTCVSYKYFSSIRRCNCPKKFGGEHCEIDK
SKTCYEGNGHFYRGKASTDTMGRPCLPWNSATVLQQTYHAHRSDALQLGLGKHNYCR
NPDNRRRPWCYVQVGLKPLVQECMVHDCADGGGPSSPPEELKFQCGQKTLRPRXKII
GGEFTTIENQPWFAAIYRRHRGGSVTYVCGGSLISPCWVISATHCFIDYPKKEDYIV
YLGRSRLNSNTQGEMKFEVENLILHKDYSADTLAHHNDIALLKIRSKEGRCAQPSRT
IQTICLPSMYNDPQFGTSCEITGFGKENSTDYLYPEQLKMTVVKLISHRECQQPHYY
GSEVTTKMLCAADPQWKTDSCQGDSGGPLVCSLQGRMTLTGIVSWGRGCALKDKPGV
YTRVSHFLPWIRSHTKEENGLALSAGGAS
SEQ ID NO: 27: Amino acid sequence of 102-10 HCDR1 FTNYGMN
SEQ ID NO: 28: Amino acid sequence of 102-10 HCDR2 WINTYTGEATYA
SEQ ID NO: 29: Amino acid sequence of 102-10 HCDR3 LMDY
SEQ ID NO: 30: Amino acid sequence of 102-10 LCDR1 KASQDINKYIA
SEQ ID NO: 31: Amino acid sequence of 102-10 LCDR2 YTSTLQP
SEQ ID NO: 32: Amino acid sequence of 102-10 LCDR3 LQYDNLTW
SEQ ID NO: 33: Amino acid sequence of 34-105 HCDR1 KSVSTSGYSY
SEQ ID NO: 34: Amino acid sequence of 34-105 HCDR2 LVS
SEQ ID NO: 35: Amino acid sequence of 34-105 HCDR3 QHIRELTR
SEQ ID NO: 36: Amino acid sequence of 34-105 LCDR1 GFTFSSYA
SEQ ID NO: 37: Amino acid sequence of 34-105 LCDR2 ISSGGTT
SEQ ID NO: 38: Amino acid sequence of 34-105 LCDR3 VRGGTIGAY
SEQ ID NO: 39: Amino acid sequence of Fib-0355 HCDR1 QSVLYSSNQK
SEQ ID NO: 40: Amino acid sequence of Fib-0355 HCDR2 YWASTRES
SEQ ID NO: 41: Amino acid sequence of Fib-0355 HCDR3 YLSS SEQ ID NO: 42: Amino acid sequence of Fib-0355 LCDR1 YTFTNYG
SEQ ID NO: 43: Amino acid sequence of Fib-0355 LCDR2 NTNTGE
SEQ ID NO: 44: Amino acid sequence of Fib-0355 LCDR3 RLLDY
SEQ ID NO: 45: Amino acid sequence of 99 HCDR1 NYGMN
SEQ ID NO: 46: Amino acid sequence of 99 HCDR2 WINTKIGEPTYAEEFKG
SEQ ID NO: 47: Amino acid sequence of 99 HCDR3 LLDY
SEQ ID NO: 48: Amino acid sequence of 99 LCDR1 RASESVDSYGNSFMH
SEQ ID NO: 49: Amino acid sequence of 99 LCDR2 RASNLES
SEQ ID NO: 50: Amino acid sequence of 99 LCDR3 QQSNEDPRT
SEQ ID NO: 51: Amino acid sequence of 1101 HCDR1 SYWMH
SEQ ID NO: 52: Amino acid sequence of 1101 HCDR2 AIYPGNSDTRNNQKFKG
SEQ ID NO: 53: Amino acid sequence of 1101 HCDR3 KAHYGNYGFAY
SEQ ID NO: 54: Amino acid sequence of 1101 LCDR1 KASDHINNWLA
SEQ ID NO: 55: Amino acid sequence of 1101 LCDR2 GATSLET
SEQ ID NO: 56: Amino acid sequence of 1101 LCDR3 QQYWSTPLT
SEQ ID NO: 57: Amino acid sequence of 0211 HCDR1 SYAMS
SEQ ID NO: 58: Amino acid sequence of 0211 HCDR2 AISSGGTTYYPDSVKG
SEQ ID NO: 59: Amino acid sequence of 0211 HCDR3 GGTIGAYW
SEQ ID NO: 60: Amino acid sequence of 0211 LCDR1 KSSQSVLYSSNQKNYLA
SEQ ID NO: 61: Amino acid sequence of 0211 LCDR2 WASTRES
SEQ ID NO: 62: Amino acid sequence of 0211 LCDR3 HQYLSSWT
SEQ ID NO: 63: Amino acid sequence of AMU1114 (Δ22-28, F157E)

EVQLVQSGAEVKKPGESLKISCKGSGYSFTSYWMHWVRQMPGKGLEWIGAIYPGNSD

TRYSPSFQGQVTISADKSISTAYLQWSSLKASDTAMYYCTRKAHYGNYGFAYWGQGT

LVTVSSASTKGPSVFPLAPSSKSTSGGTAALGCLVKDYFPEPVTVSWNSGALTSGVH

TFPAVLQSSGLYSLSSVVTVPSSSLGTQTYICNVNHKPSNTKVDKKVEPKSCGSGGG

GSGGGGSGGSSSNELHQVPSNCDCLNGGTCVSGGHWCNCPKKFGGQHCEIDKSKTCY

EGNGHFYRGKASTDTMGRPCLPWNSATVLQQTYHAHRSDALQLGLGKHNYCRNPDNR

RRPWCYVQVGLKPLVQECMVHDCADGGGPSSPPEELKFQCGQKTLRPREKIIGGEFT

TIENQPWFAAIYRRHRGGSVTYVCGGSLISPCWVISATHCFIDYPKKEDYIVYLGRS

RLNSNTQGEMKFEVENLILHKDYSADTLAHHNDIALLKIRSKEGRCAQPSRTIQTIC

LPSMYNDPQFGTSCEITGFGKENSTDYLYPEQLKMTVVKLISHRECQQPHYYGSEVT

TKMLCAADPQWKTDSCQGDSGGPLVCSLQGRMTLTGIVSWGRGCALKDKPGVYTRVS

HFLPWIRSHTKEENGLALSAGGAS

## Claims

1. A fusion protein comprising an antibody that binds to insoluble fibrin or an antigen-binding fragment thereof and a catalytic domain of prourokinase having an amino acid sequence corresponding to a sequence of amino acids at positions 156 to 411 of prourokinase set forth in SEQ ID NO: 6 or 7, wherein the antibody or an antigen-binding fragment thereof and the catalytic domain of prourokinase are linked directly or via a linker to each other, wherein
the antibody or an antigen-binding fragment thereof contains a heavy chain variable region and light chain variable region of the antibody that binds to insoluble fibrin, and
phenylalanine of the catalytic domain corresponding to phenylalanine at position 157 of the prourokinase set forth in SEQ ID NO: 6 or 7 is substituted with another amino acid.

2. The fusion protein according to claim 1, wherein the phenylalanine is substituted with isoleucine, glutamic acid, aspartic acid or cysteine.

3. The fusion protein according to claim 1 or 2,
wherein the phenylalanine is substituted with glutamic acid or aspartic acid.

4. The fusion protein according to any one of claims 1 to 3, wherein
binding ability to one or more urokinase receptor selected from the group consisting of a human urokinase receptor and a mouse urokinase receptor is lower than binding ability of a fusion protein having an amino acid sequence set forth in SEQ ID NO: 1.

5. The fusion protein according to any one of claims 1 to 4, wherein
the fusion protein shows no significant binding to one or more urokinase receptor selected from the group consisting of a human urokinase receptor and a mouse urokinase receptor.

6. The fusion protein according to any one of claims 1 to 3, further comprising
an EGF-like domain having an amino acid sequence corresponding to a sequence of amino acids at positions 7 to 43 of the prourokinase set forth in SEQ ID NO: 6 or 7 between the antibody or an antigen-binding fragment portion thereof and the catalytic domain, wherein
amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are each substituted with another amino acid, thereby lowering or eliminating binding ability to a human urokinase receptor.

7. The fusion protein according to any one of claims 1 to 4, further comprising
an EGF-like domain having an amino acid sequence corresponding to a sequence of amino acids at positions 7 to 43 of the prourokinase set forth in SEQ ID NO: 6 or 7 between the antibody or an antigen-binding fragment portion thereof and the catalytic domain, wherein
amino acids of the EGF-like domain corresponding to asparagine at position 22, asparagine at position 27, histidine at position 29, tryptophan at position 30 and glutamine at position 40 of the prourokinase set forth in SEQ ID NO: 6 or 7 are substituted with tyrosine, serine, arginine, arginine and glutamic acid, respectively, thereby lowering or eliminating binding ability to a human urokinase receptor.

8. The fusion protein according to any one of claims 1 to 4, further comprising
an EGF-like domain having an amino acid sequence corresponding to a sequence of amino acids at positions 7 to 43 of the prourokinase set forth in SEQ ID NO: 6 or 7 between the antibody or an antigen-binding fragment portion thereof and the catalytic domain, wherein
amino acids of the EGF-like domain corresponding to the amino acids from asparagine at position 22 to isoleucine at position 28 of the prourokinase set forth in SEQ ID NO: 6 or 7 are deleted.

9. A pharmaceutical formulation comprising the fusion protein according to any one of claims 1 to 8.

10. A method for producing the fusion protein according to any one of claims 1 to 8, comprising culturing a protein-producing cell expressibly having a nucleic acid encoding the fusion protein to express the fusion protein and purifying the fusion protein from a culture obtained.

11. The method according to claim 10, wherein the culturing is performed in a serum-free condition or in the presence of 5% or less of serum.

12. The method according to claim 10 or 11, wherein the purifying is performed in the absence of L-arginine.

13. A method for producing a fusion-protein variant excellent in stability or a pharmaceutical composition containing the fusion-protein variant, wherein
the fusion protein comprises
an antibody that binds to insoluble fibrin or an antigen-binding fragment thereof and a catalytic domain of prourokinase having an amino acid sequence corresponding to a sequence of amino acids at positions 156 to 411 of prourokinase set forth in SEQ ID NO: 6 or 7, wherein the antibody or an antigen-binding fragment thereof and the catalytic domain of prourokinase are linked directly or via a linker to each other;
the antibody or antigen-binding fragment thereof contains a heavy chain variable region and light chain variable region of the antibody that binds to insoluble fibrin; and
the method comprising introducing an F157E or F157D mutation to the fusion protein, thereby imparting excellent stability compared to the fusion protein not modified.
